# EUROPÄISCHE PATENTANMELDUNG

(11) **EP 1 310 238 A2**
(43) Veröffentlichungstag der Anmeldung: **14.05.2003**
(21) Anmeldenummer: 02025192.2
(22) Anmeldetag: 11.11.2002
(51) Int. Cl.: A61K 7/42

(54) **Kosmetische oder dermatologische Lichtschutzmittelzubereitung**

(30) Priorität: 12.11.2001 DE 10155542
(71) Anmelder: BASF AKTIENGESELLSCHAFT, 67056 Ludwigshafen (DE)
(72) Erfinder: Parker, Robert, 68161 Mannheim (DE); Heidenfelder, Thomas, 67125 Dannstadt (DE); Wagenblast, Gerhard, 67157 Wachenheim (DE)
(74) Vertreter: Pohl, Michael, Dr.

(57) **Zusammenfassung**

Beschrieben werden kosmetische oder dermatologische Zubereitungen für Haut und Haare, die wenigstens eine im infraroten Wellenlängenbereich von 750 nm bis 2500 nm reflektierende cholesterischflüssigkristalline Komponente, wenigstens eine vor Strahlung im ultravioletten Strahlenbereich von 280 nm bis 450 nm schützende Komponente und einen kosmetisch akzeptablen Träger enthalten, sowie deren Verwendung zur Reduzierung der Erwärmung von Haut und Haaren und zum Schutz gegen Sonnenstrahlen.

## Beschreibung

Die Erfindung betrifft kosmetische oder dermatologische Lichtschutzmittelzubereitungen, die wenigstens eine im infraroten Wellenlängenbereich von 750 nm bis 2500 nm reflektierende cholesterisch-flüssigkristalline Komponente, wenigstens eine vor Strahlung im ultravioletten Strahlenbereich von 280 nm bis 449 nm schützende Filtersubstanz und wenigstens einen kosmetisch akzeptablen Träger enthalten sowie deren Verwendung.

Die in kosmetischen und dermatologischen Zubereitungen eingesetzten Lichtschutzmittel haben die Aufgabe, schädigende Einflüsse des Sonnenlichts auf die menschliche Haut zu verhindern oder zumindest in ihren Auswirkungen zu reduzieren. Daneben dienen diese Lichtschutzmittel aber auch dem Schutz weiterer Inhaltsstoffe vor Zerstörung oder Abbau durch UV-Strahlung. In haarkosmetischen Formulierungen soll eine Schädigung der Keratinfaser durch UV-Strahlen vermindert werden.

Das an die Erdoberfläche gelangende Sonnenlicht hat einen Anteil an UV-B- (280 bis 320 nm) und an UV-A-Strahlung (oberhalb 320 nm), welche sich direkt an den Bereich des sichtbaren Lichtes anschließen. Der Einfluss auf die menschliche Haut macht sich besonders bei der UV-B-Strahlung durch Sonnenbrand bemerkbar. Dementsprechend bietet die Industrie eine größere Zahl von Substanzen an, welche die UV-B-Strahlung absorbieren und damit den Sonnenbrand verhindern.

Häufig enthalten die Lichtschutzmittel mehrere im UV-Bereich absorbierende Verbindungen, um einen möglichst weitreichenden Schutz im UV-A- und UV-B-Bereich zu erzielen. Außerdem müssen Lichtschutzmitel für kosmetische Zwecke noch eine Vielzahl weiterer Anforderungen erfüllen, beispielsweise gute Löslichkeit in kosmetischen Ölen, hohe Stabilität der mit ihnen hergestellten Emulsionen, toxikologische Unbedenklichkeit sowie geringen Eigengeruch und geringe Eigenfärbung.

Die DE-A-197 45 647 beschreibt Wärmeisolationsbeschichtungen für den Automobil- und Bausektor, die wenigstens eine cholesterische IR-reflektierende Schicht umfassen.

Die DE-A-198 48 130 und die DE-A-198 24 972 offenbaren die Verwendung von cholesterischen Pigmenten als UV-Filter in kosmetischen und pharmazeutischen Zubereitungen.

Die DE-A-196 297 61 beschreibt kosmetische oder pharmazeutische Zubereitungen, die Pigmente mit vom Betrachtungswinkel abhängiger Farbigkeit enthalten. Die Pigmente enthalten mindestens eine orientierte vernetzte Substanz mit flüssigkristalliner Struktur und mit chiraler Phase. Die hier in den kosmetischen und pharmazeutischen Rezepturen eingesetzten Pigmente haben den Nachteil, dass es sich um farbige Verbindungen handelt, deren Einsatzmöglichkeit in kosmetischen und pharmazeutischen Zubereitungen, mit denen ein UV-Schutz erzielt wird, nur begrenzt möglich ist.

Die WO-A-99/11733 beschreibt ganz allgemein eine Eignung farbiger cholesterischer Pigmente für Anwendungen im Kosmetikbereich. Ein Einsatz in Sonnenschutzmitteln zum Schutz vor IR- und UV-Strahlung ist jedoch nicht beschrieben.

Die bisher üblichen Lichtschutzmittel verhindern nicht, dass sich Haut und Haare unter dem Einfluss der Sonnenstrahlung spürbar erwärmen. Dies wird in jüngster Zeit zunehmend als störend empfunden; man fühlt sich nicht mehr frisch. Außerdem wurde gefunden, dass die Wärmestrahlung eine Schädigung der Haut und/oder der Haare hervorrufen kann, beispielsweise werden Lichturtikaria durch Strahlung im infraroten Wellenlängenbereich mitausgelöst. Es besteht daher ein aktueller Bedarf an Lichtschutzmitteln, welche die Haut und die Haare vor Wärmeeinwirkung schützen.

Der vorliegenden Erfindung liegt daher die Aufgabe zugrunde, leicht herstellbare, kosmetische oder dermatologische Lichtschutzmittelzubereitungen bereitzustellen, die einen effektiven Schutz vor UV- und Wärmestrahlung bieten. Die Lichtschutzmittel sollen vorzugsweise mit hoher Extinktion absorbieren, photostabil sein und keine oder nur eine geringe Eigenfarbe im sichtbaren Bereich, d. h. eine scharfe Bandenstruktur, aufweisen.

Erfindungsgemäß wird die Aufgabe durch eine kosmetische oder dermatologische Zubereitung gelöst, die umfasst
A) wenigstens eine im infraroten Wellenlängenbereich von 750 nm bis 2500 nm reflektierende cholesterisch-flüssigkristalline Komponente,
B) wenigstens eine vor Strahlung im ultravioletten Strahlenbereich von 280 nm bis 449 nm schützende Komponente und
C) einen kosmetisch oder dermatologisch akzeptablen Träger.

Die erfindungsgemäße Zubereitung umfasst als Bestandteil A) eine cholesterisch-flüssigkristalline Komponente. Voraussetzung für das Auftreten cholesterischer Phasen ist die Chiralität. Der chirale Molekülteil kann sowohl im flüssigkristallinen Molekül selbst vorgegeben sein, als auch als Dotierstoff zu einer nematischen Phase gegeben werden, wodurch eine chiral nematische Phase induziert wird. Grundsätzlich lassen sich nahezu alle bekannten cholesterischen Monomere bzw. Monomergemische oder Polymere bzw. Polymergemische durch Variation der chiralen Komponente in der Ganghöhe ihrer helikalen Überstruktur so einstellen, dass ihr Reflexionsmaximum in einem bestimmten Bereich des elektromagnetischen Spektrums liegt.

Bevorzugt ist die Komponente A) ausgewählt unter
a) mindestens einem cholesterischen polymerisierbaren Monomer;
b) mindestens einem achiralen, nematischen, polymerisierbaren Monomer und einer chiralen Verbindung;
c) mindestens einem cholesterischen vernetzbaren Polymer;
d) mindestens einem cholesterischen Polymer in einem polymerisierbaren Verdünnungsmittel oder einem Gemisch polymerisierbarer Verdünnungsmittel;
e) mindestens einem cholesterischen Polymer, dessen cholesterische Phase durch schnelles Abkühlen unter die Glasübergangstemperatur eingefroren werden kann; oder
f) mindestens einem achiralem, flüssigkristallinen vernetzbaren Polymer und einer chirale Verbindung;
jeweils in gehärtetem Zustand.

Im Rahmen der vorliegenden Erfindung wird unter Vernetzung die kovalente Verknüpfung polymerer Verbindungen und unter Polymerisation die kovalente Verknüpfung monomerer Verbindungen zu Polymeren verstanden. Unter Härtung wird Vernetzung, Polymerisation oder das Einfrieren einer cholesterischen Phase verstanden.

Durch Härtung wird die gleichmäßige Orientierung der cholesterischen Moleküle in der Cholesterschicht fixiert.

Geeignete cholesterische Komponenten A) sind in der DE-A-197 45 647 beschrieben, auf die hiermit im vollen Umfang Bezug genommen wird.

Bevorzugt unter den zuvor genannten Ausführungsformen a) bis f) sind die Varianten a) und b).

### Variante a):

Insbesondere umfassen die Monomere a) mindestens ein chirales, flüssigkristallines, polymerisierbares Monomer der Formel I 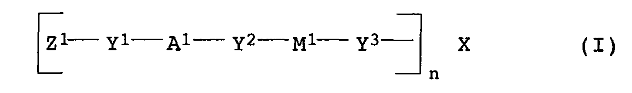 in der die Variablen die folgende Bedeutung haben:
- Z¹: eine polymerisierbare Gruppe oder ein Rest, der eine polymerisierbare Gruppe trägt,
- Y¹,Y²,Y³: unabhängig voneinander chemische Bindungen, Sauerstoff, Schwefel,
―CO―O―, ―O―CO―, ―O―CO―O―,
―CO―S― , ―S―CO― ,
―CO―N(R)― , ―N(R)―CO― , CH₂O, OCH₂ ,
―N(R)―CO―O― oder ―O―CO― N(R)― ,
- A¹: ein Spacer,
- M¹: eine mesogene Gruppe,
- X: ein n-wertiger chiraler Rest,
- R: Wasserstoff oder C₁-C₄-Alkyl,
- n: 1 bis 6
wobei die Reste Z¹, Y¹, Y², Y³, A¹ und M¹, gleich oder verschieden sein können, wenn n größer als 1 ist.

Bevorzugte Reste Z¹ sind:
CH₂= CH-, CH ≡ C-, 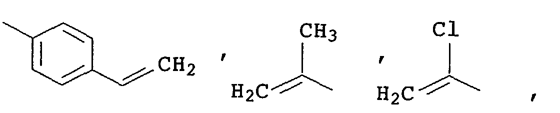 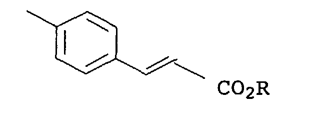 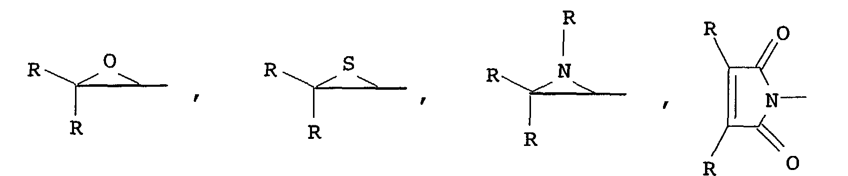 ―N=C=O, ―N=C=S, ―O―C≡N,
―COOH, ― OH oder ― NH₂,
wobei die Reste R gleich oder verschieden sein können und Wasserstoff oder C₁-C₄-Alkyl, wie Methyl, Ethyl, n-Propyl, iso-Propyl, n-Butyl, iso-Butyl, sec.-Butyl oder tert.-Butyl, bedeuten. Von den reaktiven polymerisierbaren Gruppen können die Cyanate spontan zu Cyanuraten trimerisieren und sind daher bevorzugt. Die anderen genannten Gruppen benötigen zur Polymerisation weitere Verbindungen mit komplementären reaktiven Gruppen. So können beispielsweise Isocyanate mit Alkoholen zu Urethanen und mit Aminen zu Harnstoffderivaten polymerisieren. Analoges gilt für Thiirane und Aziridine. Carboxylgruppen können zu Polyestern und Polyamiden kondensiert werden. Die Maleinimidogruppe eignet sich besonders zur radikalischen Copolymerisation mit olefinischen Verbindungen wie Styrol. Die komplementären reaktiven Gruppen können dabei entweder in einer zweiten Verbindung vorhanden sein, die mit der ersteren gemischt wird, oder sie können durch Hilfsverbindungen, die 2 oder mehr dieser komplementären Gruppen enthalten, in das polymere Netzwerk eingebaut werden.

Besonders bevorzugte Gruppierungen Z¹-Y¹ sind Acrylat und Methacrylat.

Repräsentative Spacer A¹ sind beispielsweise:
-(CH₂)ₚ-, -(CH₂CH₂O)ₘCH₂CH₂-, -CH₂CH₂SCH₂CH₂-, -CH₂CH₂NHCH₂CH₂-, 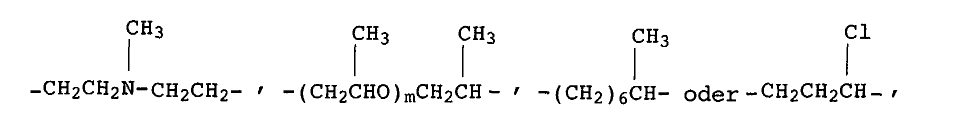 wobei
- m: für 1 bis 3 und
- p: für 1 bis 12 steht.

Die mesogene Gruppe M¹ hat vorzugsweise die Struktur

(T-Y⁸)ₛ-T

wobei Y⁸ ein Brückenglied gemäß einer der Definitionen von Y¹, s eine Zahl von 1 bis 3 und T gleiche oder verschiedene zweiwertige isocycloaliphatische, heterocycloaliphatische, isoaromatische oder heteroaromatische Reste bedeuten. Wenn s für eine Zahl >1 steht, können die Brückenglieder Y⁸ gleich oder verschieden sein.

Die Reste T können auch durch C₁-C₄-Alkyl, Fluor, Chlor, Brom, Cyan, Hydroxy oder Nitro substituierte Ringsysteme sein.

Besonders bevorzugt sind die folgenden mesogenen Gruppen M¹:

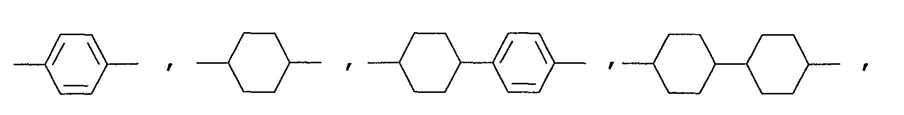

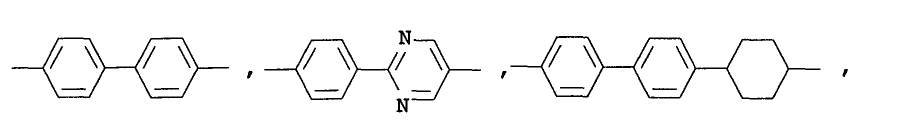

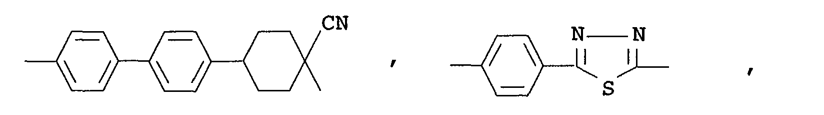

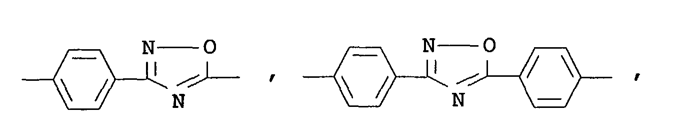

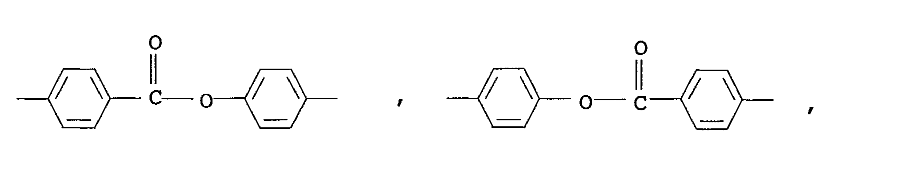

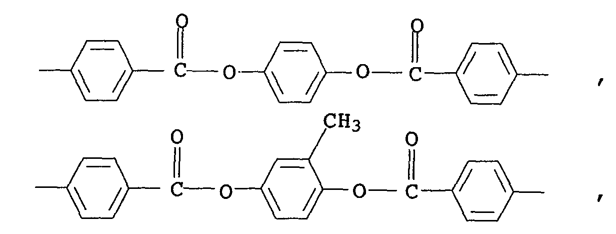

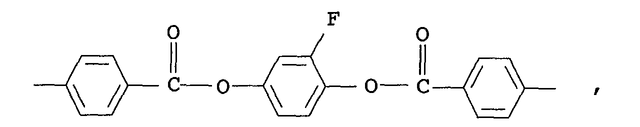

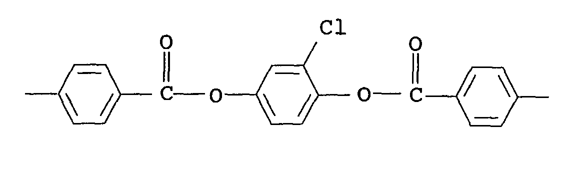

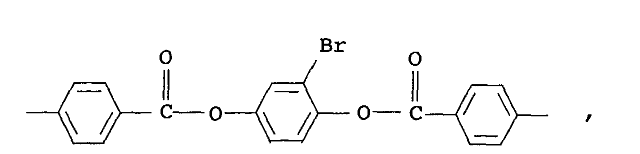

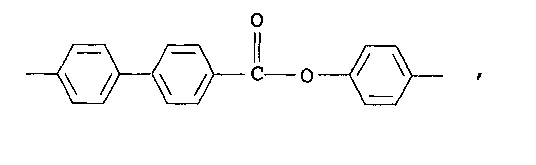

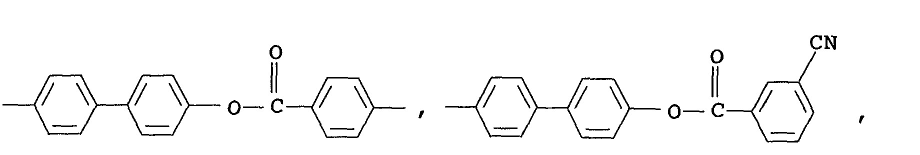

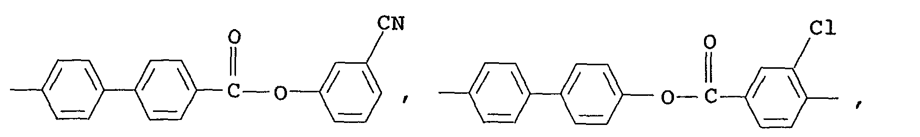

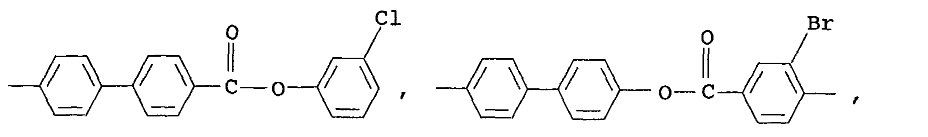

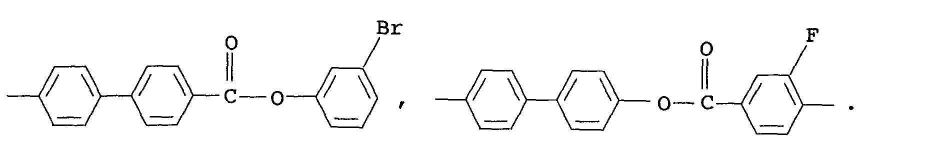

Von den chiralen Resten X der Verbindungen der Formel I sind u.a. aufgrund der Verfügbarkeit insbesondere solche bevorzugt, die sich von Zuckern, Binaphthyl- oder Biphenylderivaten sowie optisch aktiven Glykolen, Dialkoholen oder Aminosäuren ableiten. Bevorzugte Zucker sind insbesondere Pentosen und Hexosen und davon abgeleitete Derivate.

Beispiele für Reste X sind die folgenden Strukturen, wobei die endständigen Striche jeweils die freien Valenzen bedeuten.

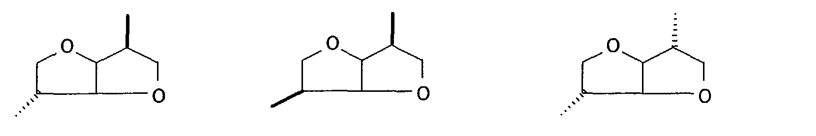

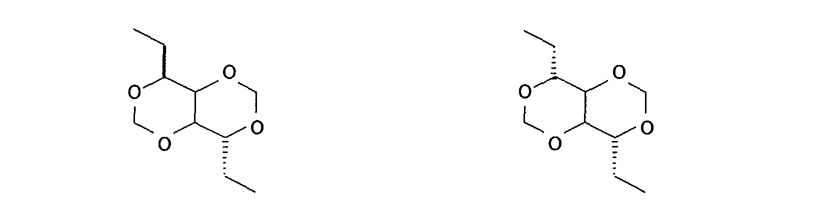

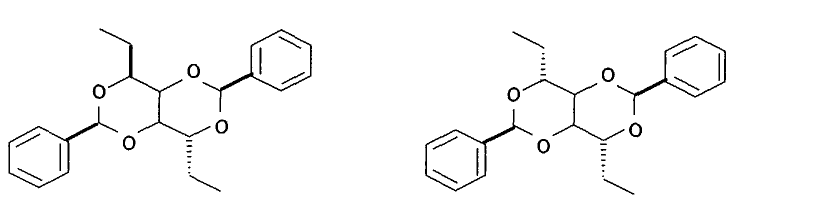

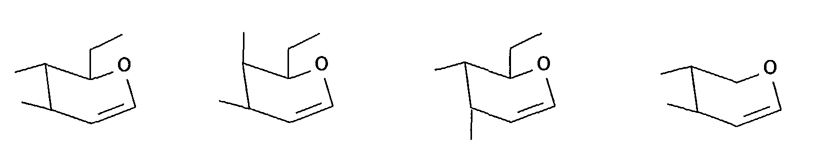

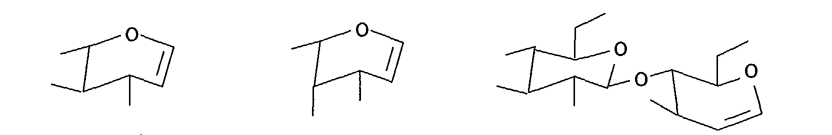

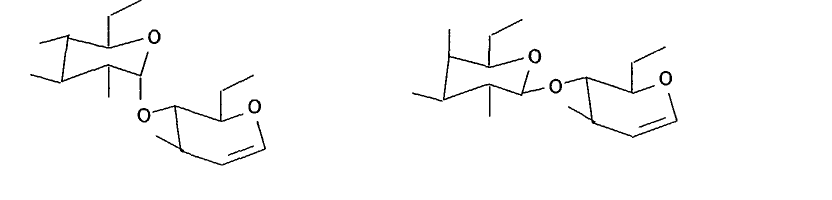

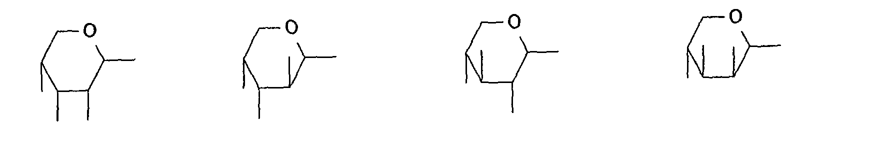

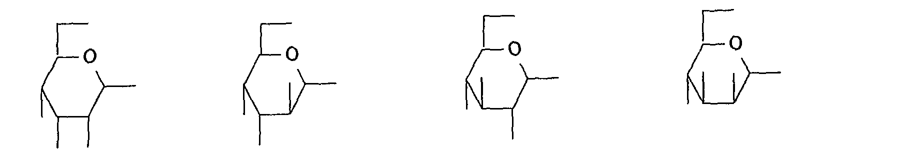

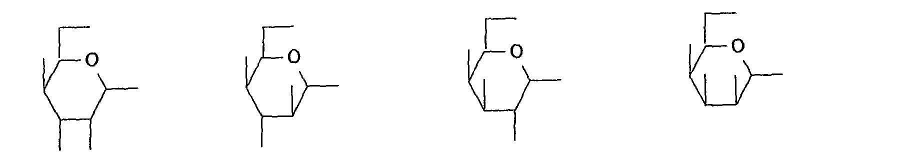

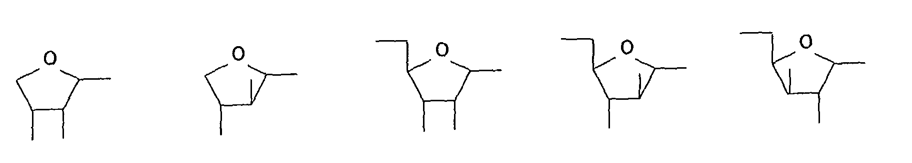

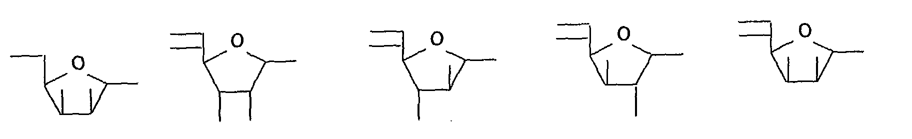

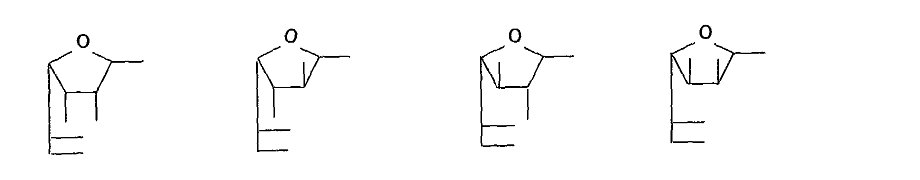

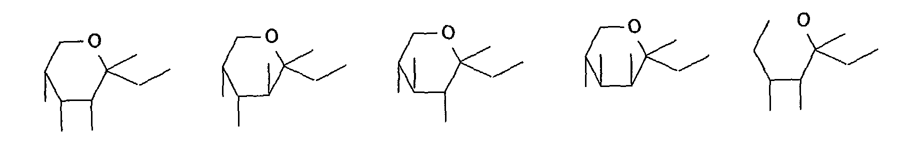

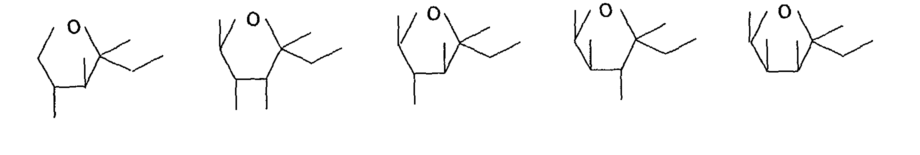

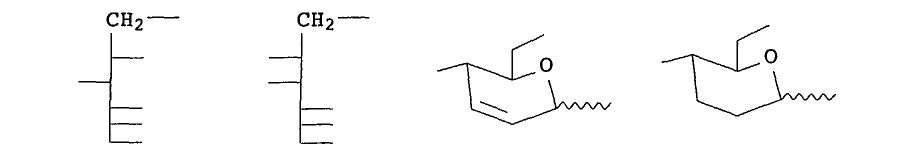

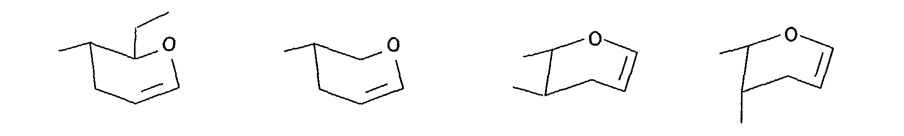

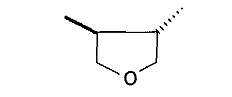

Besonders bevorzugt sind 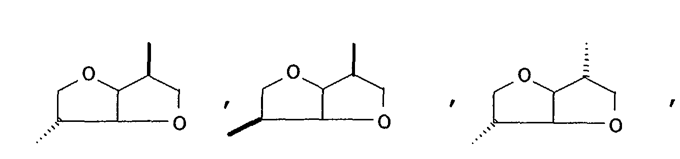 und

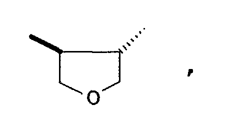

Weiterhin sind auch chirale Gruppen geeignet, die folgende Strukturen aufweisen:

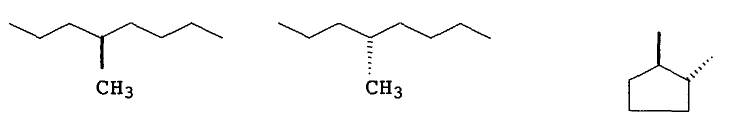

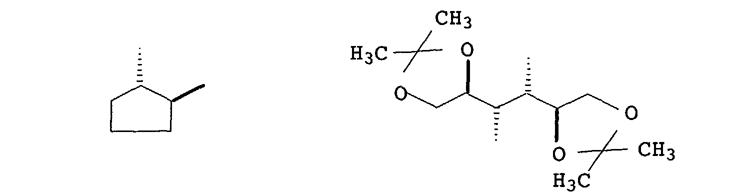

Weitere Beispiele sind in der deutschen Anmeldung P 43 42 280.2 aufgeführt.

n ist bevorzugt 2.

### Variante b):

Als bevorzugte Monomere der Gruppe b) enthält das polymerisierbare Gemisch mindestens ein achirales nematisches, polymerisierbares Monomer der Formel II

Z²―Y⁴―A²―Y⁵―M²―Y⁶―A³(̵Y⁷―Z³)_{d} (II)

in der die Variablen die folgende Bedeutung haben:
- Z², Z³: gleiche oder verschiedene polymerisierbare Gruppen oder Reste, die eine polymerisierbare Gruppe enthalten
- d: 0 oder 1
- Y⁴, Y⁵, Y⁶, Y⁷: unabhängig voneinander chemische Bindungen, Sauerstoff, Schwefel,
―CO―O―,―O―CO― ,―O―CO―O― ,
―CO―S― , ―S―CO― ,
―CO―N(R)― , ―N(R)―CO― , CH₂O, OCH₂,
― N(R)―CO―O― oder ―O―CO― N(R)―
- A², A³: gleiche oder verschiedene Spacer und
- M²: eine mesogene Gruppe.

Dabei gelten für die polymerisierbaren Gruppen, die Brückenglieder Y⁴ bis Y⁷, die Spacer und die mesogene Gruppe die gleichen Bevorzugungen wie für die entsprechenden Variablen der Formel I.

Außerdem enthält das Gemisch der Gruppe b) eine chirale Verbindung. Die chirale Verbindung bewirkt die Verdrillung der achiralen flüssigkristallinen Phase zu einer cholesterischen Phase. Dabei hängt das Ausmaß der Verdrillung von der Verdrillungsfähigkeit des chiralen Dotierstoffs und von seiner Konzentration ab. Damit hängt also die Ganghöhe der Helix und wiederum auch die Reflexionswellenlänge von der Konzentration des chiralen Dotierstoffs ab. Es kann daher für den Dotierstoff kein allgemeingültiger Konzentrationsbereich angegeben werden. Der Dotierstoff wird in der Menge zugegeben, bei der die gewünschte Reflexion entsteht.

Bevorzugte chirale Verbindungen sind solche der Formel Ia 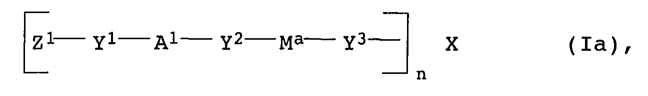 in der Z¹, Y¹, Y², Y³, A¹, X und n die obengenannten Bedeutungen haben und M^{a} ein zweiwertiger Rest ist, der mindestens ein heterooder isocyclisches Ringsystem enthält.

Der Molekülteil M^{a} ähnelt dabei den beschriebenen mesogenen Gruppen, da auf diese Weise eine besonders gute Kompatibilität mit der flüssigkristallinen Verbindung erreicht wird. M^{a} muß jedoch nicht mesogen sein, da die Verbindung Ia lediglich durch ihre chirale Struktur eine entsprechende Verdrillung der flüssigkristallinen Phase bewirken soll. Bevorzugte Ringsysteme, die in M^{a} enthalten sind, sind die oben erwähnten Strukturen T, bevorzugte Strukturen M^{a} solche der oben genannten Formel (T-Y⁸)ₛ-T. Weitere Monomere und chirale Verbindungen der Gruppe b) sind in der WO 97/00600 beschrieben, auf die hiermit in vollem Umfang Bezug genommen wird.

Besonders bevorzugt enthält die erfindungsgemäße Zubereitung chirale Verbindungen (2A) bis (2E) in all ihren diastereomeren Ausführungsformen: 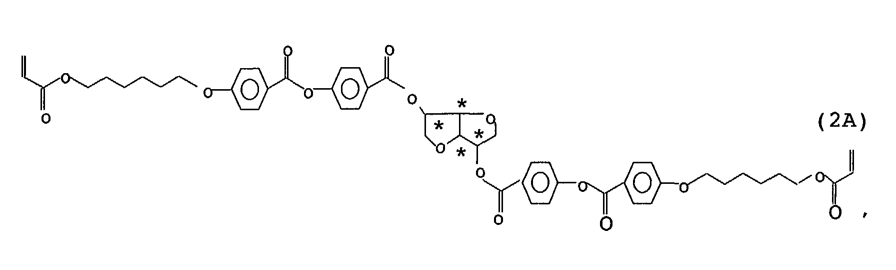 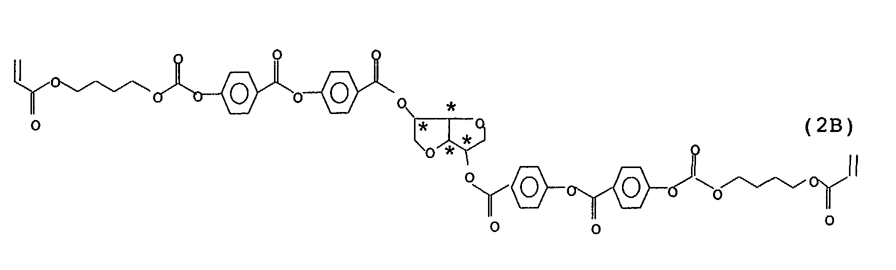 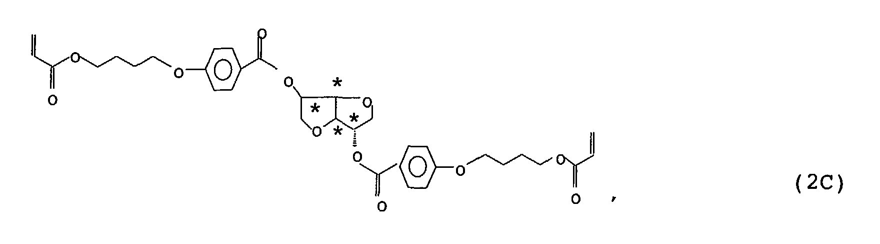 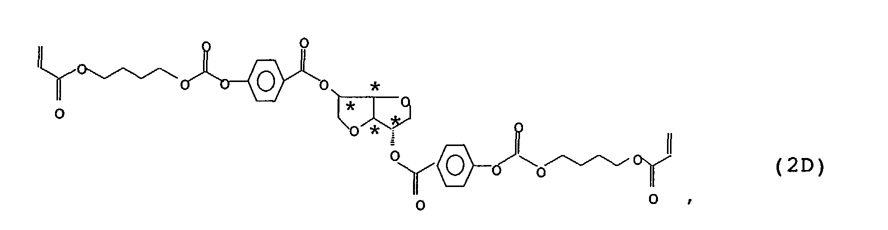 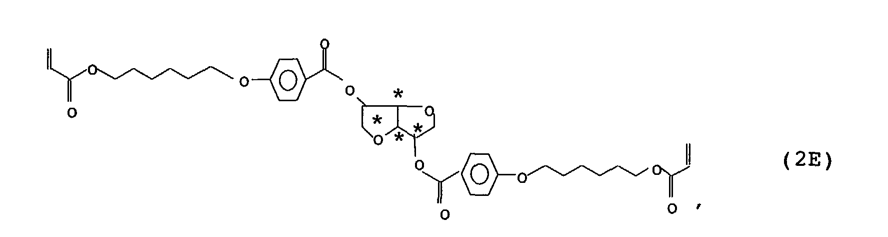 worin * ein asymmetrisches Kohlenstoffatom bedeutet
und nematische Monomere der Formel 1: 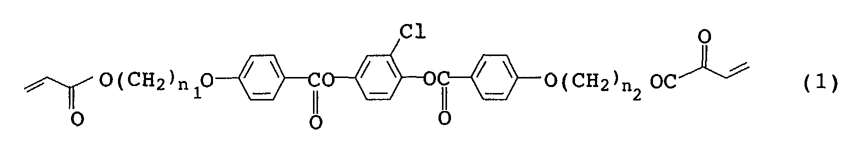 der Formel 3: 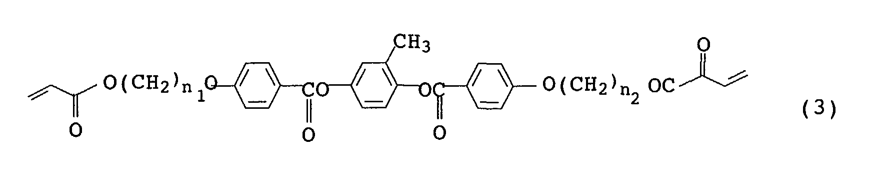 oder der Formel 4: 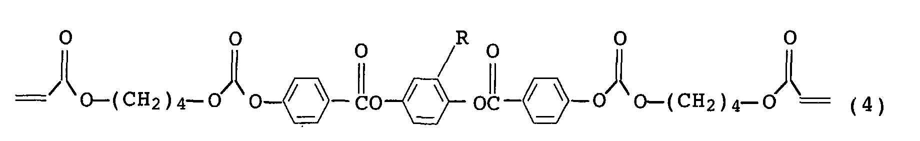 wobei in den Formeln 1 und 3 n₁ und n₂ unabhängig voneinander für 2, 4 oder 6 stehen und die Monomeren der Formel 1 oder 3 vorzugsweise als Gemische von Verbindungen mit n₁/n₂ = 2/4, 2/6, 4/2, 6/2, 4/4, 4/6, 6/4 oder 6/6 eingesetzt werden, und R in Formel 4 für H, Cl oder CH₃ steht.

Bevorzugt wird die Komponente A) in Form von Pigmenten eingesetzt. Zur Herstellung der Pigmente überführt man die in der cholesterischen Komponente enthaltenen Monomere bzw. polymerisierbaren oder vernetzbaren Gruppen durch radikalische oder ionische Polymerisationsverfahren, welche durch eine thermische oder photochemische Reaktion gestartet werden können, in hochvernetzte Polymere mit eingefrorener flüssigkristalliner Ordnungsstruktur.

Die Herstellung solcher Pigmente ist bekannt und wird u.a. ausführlich in der WO 99/11733 und in der DE-A-197 38 369 beschrieben.

In einer bevorzugten Ausführungsform werden dreidimensional vernetzbare polymerisierbare Monomere auf eine Unterlage aufgebracht, auf dieser Unterlage vernetzt und nach dem Vernetzen von der Unterlage gelöst. Bevorzugt wird das cholesterische Material in der erfindungsgemäßen Zusammensetzung in Form eines feinteiligen Pulvers eingesetzt. Fallen die Polymere nicht bereits bei der Herstellung als feinteiliges Pulver an, so können sie nach dem Fachmann bekannten Verfahren zerkleinert werden. Bevorzugt ist eine zweistufige Zerkleinerung, wobei der erste Zerkleinerungsschritt sich an eine Strang- oder Bandextrusion anschließt. Die dabei erhaltenen Stränge oder Bänder lassen sich in bekannter Weise mit Schnitzlern oder Granulatoren in Chips oder Stranggranulate überführen.

Zur weiteren Zerkleinerung eignen sich bekannte Mahlaggregate aller Arten und Ausführungsformen. Je nach der erwünschten Anwendung bzw. je nach Art der kosmetischen oder pharmazeutischen Formulierung können Korngrößen mit einem Durchmesser von 1 bis 1000 µm hergestellt werden. Bevorzugte Korngrößen liegen im Bereich zwischen 1 und 100 µm, besonders bevorzugt zwischen 15 und 50 µm.

Die Dicke der Pigmente liegt zwischen 0,5 und 100 µm, bevorzugt zwischen 1 und 50 µm, besonders bevorzugt zwischen 1,0 und 10 µm.

Ganz besonders bevorzugt sind sogenannte Breitbandreflektoren, die sich durch Mischen mehrerer cholesterisch-flüssigkristalliner Pigmente mit jeweils unterschiedlichen IR-Reflexionsmaxima erzeugen lassen.

Darüber hinaus können Pigmente unterschiedlicher Händigkeit erhalten werden, indem man zwei verschiedene Pigmenten des cholesterischen Materials mit jeweils entgegengesetzter Verdrillung (Helicität) mischt. Pigmente mit jeweils entgegengesetzt verdrillten, cholesterischen Strukturen sind beispielsweise durch Zugabe jeweils der einzelnen Enantiomeren oder Diasteromeren der chiralen Zusatzstoffe zu den achiralen flüssigkristallinen polymerisierbaren Monomeren erhältlich. Die Ganghöhe der jeweils entgegengesetzt verdrillten Strukturen kann dabei gleich oder verschieden sein. Pigmente unterschiedlicher Händigkeit reflektieren deutlich mehr Strahlung als Pigmente gleicher Händigkeit.

Es ist auch möglich, zunächst die cholesterischen Komponenten jeweils von entgegengesetzter Verdrillung zu mischen, diese anschließend durch o. g. Härtung in die bereits beschriebenen Pigmente zu überführen und als IR-Reflektoren in kosmetischen und dermatologischen Mitteln einzusetzen.

Neben den oben genannten Mischungen cholesterisch flüssigkristalliner Pigmente ist es auch möglich Mehrschichtpigmente zu erzeugen, deren einzelne Schichten verschiedene, dreidimensional vernetzte cholesterisch-flüssigkristalline Komponenten enthalten. Das Design derartiger Mehrschichtpigmente lässt sich vielfältig variieren. So können u.a.
- einzelne Schichten von vernetzten cholesterischem Material entgegengesetzter Verdrillung oder
- einzelne Schichten von vernetzten cholesterisch-flüssigkristallinem Material gleicher Gangrichtung aber unterschiedlicher Ganghöhe und somit unterschiedlicher Reflektionseigenschaften
übereinander aufgebracht werden.

Einzelheiten bezüglich Herstellung solcher mehrschichtiger cholesterischen Pigmente sind der DE-A-197 38 368 zu entnehmen.

Durch geeignete Wahl der Zusammensetzung der Pigmente lässt sich die gewünschte Reflektion im infraroten Wellenlängenbereich erzielen, ohne dass eine Farbigkeit (im sichtbaren Bereich) gezeigt wird.

Die Pigmente lassen sich durch einfaches Abmischen in die kosmetischen und pharmazeutischen Zubereitungen einarbeiten. Es kommt dabei zu keiner Aggregation oder Separation der Pigmentteilchen.

Die erfindungsgemäße Lichtschutzmittelzubereitung enthält als Bestandteil B) wenigstens eine vor Strahlung im ultravioletten Strahlenbereich von 280 nm bis 449 nm schützende Komponente. Unter einer vor UV-Strahlung schützenden Komponente versteht man eine Substanz (UV-Filtersubstanz), die Strahlung im ultravioletten Strahlenbereich absorbiert und/oder reflektiert. Vorzugsweise umfasst der Bestandteil B) mehrere Verbindungen, die im UV-Aund/oder UV-B-Bereich absorbieren und/oder reflektieren, um die Haut oder die Haare vor dem gesamten Bereich der ultravioletten Strahlung zu schützen.

In einer bevorzugten Ausführungsform ist der Bestandteil B) ausgewählt unter folgenden Klassen: cholesterischen Komponenten mit einer Ganghöhe kleiner 450 nm, Aminobenzoesäuren, Aminobenzoesäureestern, Bicyclo[2.2.1]heptan-2-onen, Hydroxybenzoesäureestern und -salzen, Benzophenonen, Benzimidazolen, Zimtsäureestern, 3,3-Diphenylacrylsäureestern, 3-Imidazol-4-yl-acrylsäuren, 3-Imidazol-4-yl-acrylsäureestern, alicyclischen Dienonen, Triazinen, Phenylglyoxylsäuren und -salzen, Dibenzoylmethanen, 4,4'-Diarylbutadienen, Benzotriazolen, Organosiloxanbenzalmalonaten, Enaminen, cyclischen Iminoestern, anorganischen Pigmenten und Mischungen davon.

Geeignete cholesterische Materialien mit einer Ganghöhe kleiner 450 nm sind cholesterische Komponenten entsprechend den Varianten a) bis f) der zuvor genannten, im infraroten Wellenlängenbereich reflektierenden Komponenten, mit der Maßgabe, dass die Ganghöhe der cholesterisch-flüssigkristallinen Phase der Komponente B) höchstens 449 nm beträgt. Bevorzugte Komponenten sind die der Varianten a), b) und c). Besonders bevorzugte cholesterische Komponenten der Varianten a) und b) sind beispielsweise in der DE-A-198 24 972 beschrieben, auf die hiermit im vollen Umfang Bezug genommen wird.

Bevorzugte Monomere der Variante a) umfassen mindestens ein chirales, flüssigkristallines polymerisierbares Monomer der allgemeinen Formel III,

[Z⁴-Y⁹-(A⁴)ₖ-Y¹⁰-M³-Y¹¹-]ₗ-X (III)

worin die Variablen unabhängig voneinander folgende Bedeutung haben:
- A⁴: ein Spacer einer Kettenlänge von 1 bis 30 C-Atomen,
- Y⁹ bis Y¹¹: eine chemische Bindung, -O-, -S-, -C(=O)-O-, -O-C(=O)-, -CH=CH-C(=O)-O-, -O-C(=O)-O-, -C(=O)-N(R)- oder -(R)N-C(=O)-, -CH₂-O-, -O-CH₂-, -CH=N-, -N=CH-, -N=N-, -(R)N-C(=O)-O- oder -O-C(=O)-N(R)-,
- M³: eine mesogene Gruppe,
- R: Wasserstoff, C₁-C₄-Alkyl,
- Z⁴: Wasserstoff, C₁-C₄-Alkyl, eine polymerisierbare Gruppe oder ein Rest, der eine polymerisierbare Gruppe trägt,
- X: ein n-wertiger chiraler Rest,
- k: 0 oder 1,
- l: 1 bis 6,
wobei die Reste Z⁴, Y⁹, Y¹⁰, Y¹¹, A⁴ und M³ gleich oder verschieden sein können und mindestens ein Rest Z⁴ eine polymerisierbare Gruppe oder einen Rest, der eine polymerisierbare Gruppe enthält, darstellt, wenn 1 größer als 1 ist.

Bevorzugte als Komponente B) geeignete cholesterische Gemische der Variante b) umfassen mindestens ein achirales flüssigkristallines Monomer sowie eine chirale Verbindung. Diese unterscheiden sich nur hinsichtlich ihrer prozentualen, aber nicht hinsichtlich ihrer stofflichen Zusammensetzung von dem Bestandteil A) in der Ausführungsform der Variante b).

Das polymerisierbare Gemisch der Variante b) enthält vorzugsweise mindestens ein achirales flüssigkristallines polymerisierbares Monomer der Formel IV

Z⁵-Y¹³-(A⁵)ₒ-Y¹⁴-M⁴-Y¹⁵-(A⁶)_{q}-Y¹⁶-Z⁶ (IV)

worin die Variablen unabhängig voneinander folgende Bedeutung haben:
- A⁵ und A⁶: ein Spacer einer Kettenlänge von 1 bis 30 C-Atomen,
- M⁴: eine mesogene Gruppe,
- Y¹³ bis Y¹⁶: eine chemische Bindung, -O-, -S-, -C(=O)-O-, -O-C(=O)-, -CH=CH-C(=O)-O-, -O-C(=O)-O-, -C(=O)-N(R)-oder -(R)N-C(=O)-, -CH₂-O-, -O-CH₂-, -CH=N-, -N=CH-,-N=N-, -(R)N-C(=O)-O- oder -O-C(=O)-N(R)-,
- R: Wasserstoff, C₁-C₄-Alkyl,
- o und q: 0 oder 1,
- Z⁵ und Z⁶: Wasserstoff, C₁-C₄-Alkyl, eine polymerisierbare Gruppe oder ein Rest, der eine polymerisierbare Gruppe trägt, wobei mindestens eine der Variablen Z⁵ oder Z⁶ eine polymerisierbare Gruppe oder einen Rest, der eine polymerisierbare Gruppe trägt, darstellt,
und
mindestens einen chiralen Zusatzstoff.

Als chirale Dotierstoffe für flüssigkristalline Phasen sind zahlreiche Verbindungen bekannt (z.B. aus DE-A 43 42 280 und DE-A 196 11 101, sowie das zuvor Gesagte). Außerdem sollten die chiralen Dotierstoffe eine gute Kompatibilität zu den flüssigkristallinen Verbindungen zeigen, so dass eine effektive Wechselwirkung zwischen diesen Komponenten ermöglicht wird. Der Dotierstoff wird wiederum in der Menge zugegeben, mit der die gewünschte UV-Reflektion erzielt wird.

Bevorzugte chirale Zusatzstoffe sind Verbindungen der Formel V

[Z⁴-Y⁹-(A⁴)ₖ-Y¹⁰-M⁵-Y¹¹-]ₗ-X (V)

worin Z⁴, Y⁹, Y¹⁰, Y¹¹, A⁴, X, k und l die obengenannten Bedeutungen haben und M⁵ ein zweiwertiger Rest ist, der mindestens ein hetero-oder isocyclisches Ringsystem enthält.

Der Molekülteil M⁵ ähnelt dabei den beschriebenen mesogenen Gruppen, da auf diese Weise eine besonders gute Kompatibilität mit der flüssigkristallinen Verbindung erreicht wird. M⁵ muß jedoch nicht mesogen sein, da die Verbindung V lediglich durch ihre chirale Struktur eine entsprechende Verdrillung der flüssigkristallinen Phase bewirken soll. Bevorzugte Ringsysteme, die in M⁵ enthalten sind, sind die oben erwähnten Strukturen T, bevorzugte Strukturen M⁵ solche der oben genannten Formel (T-Y⁸)ₛ-T. Weitere Monomere und chirale Verbindungen der Gruppe b) sind in der WO 97/00600 und der ihr zugrundeliegenden DE-A-195 324 08 beschrieben, auf die hiermit in vollem Umfang Bezug genommen wird.

Als besonders bevorzugte Monomere IV sind folgende Strukturen zu nennen: 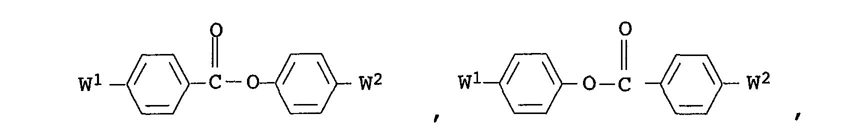 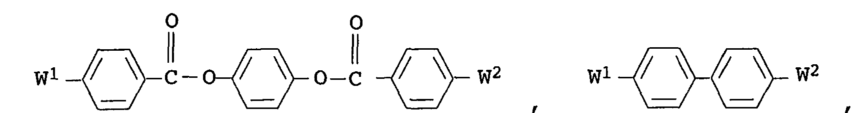 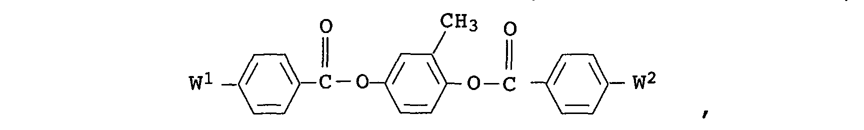 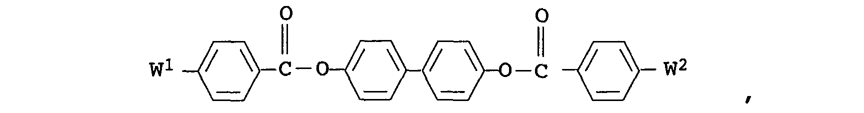 W¹: CH₂=CH-C(=O)-O-(CH₂)₄-O- , W²: -O-(CH₂)₄-O-C(=O)-CH=CH₂ 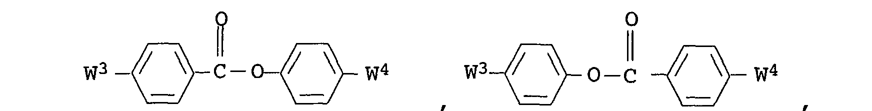 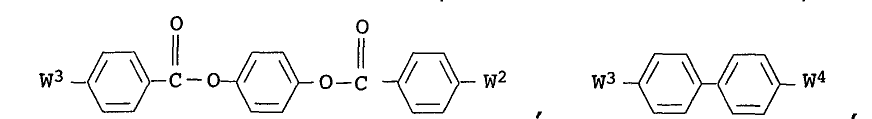 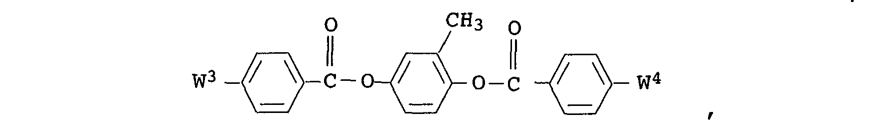 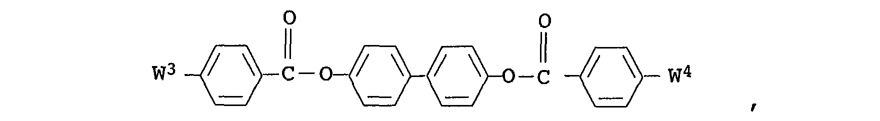 W³: CH₂=CH-C(=O)-O-(CH₂)₆-O-, W⁴: -O-(CH₂)₆-O-C(=O)-CH=CH₂ 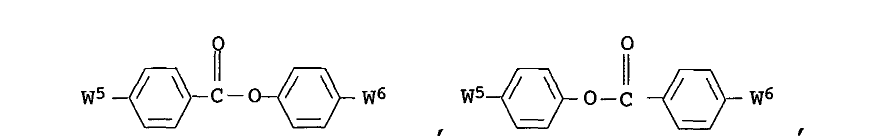 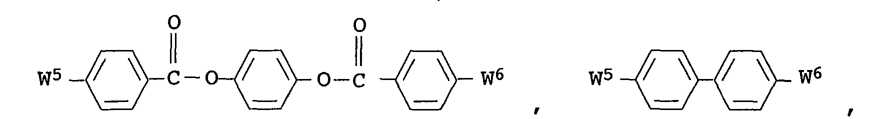 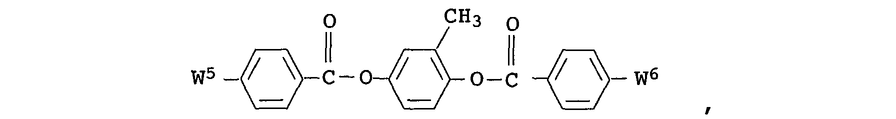 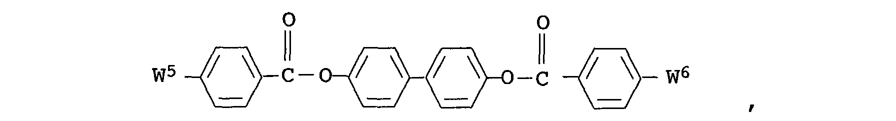 W⁵: CH₂=C(CH₃)-C(=O)-O-(CH₂)₄-O-, W⁶: -O-(CH₂)₄-O-C(=O)-C(CH₃)=CH₂ 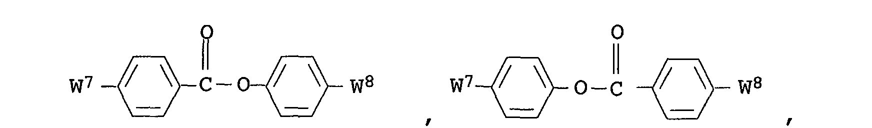 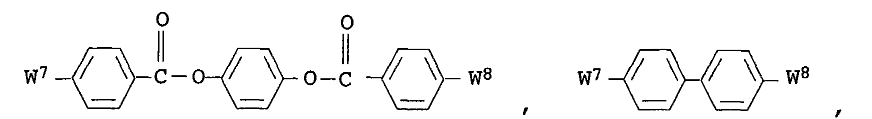 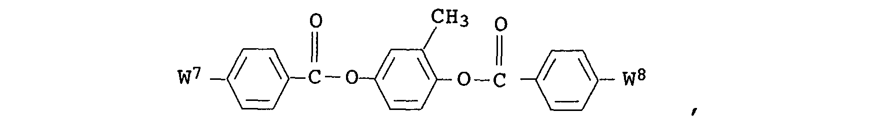 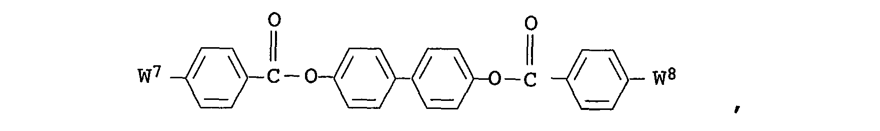 W⁷: CH₂=C(CH₃)-C(=O)-O-(CH₂)₆-O-, W⁸: -O-(CH₂)₆-O-C(=O)-C(CH₃)=CH₂ 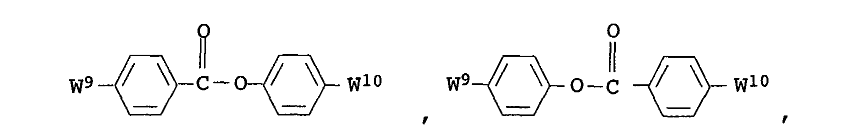 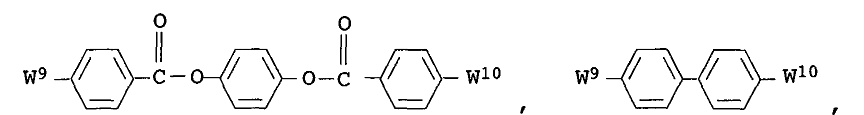 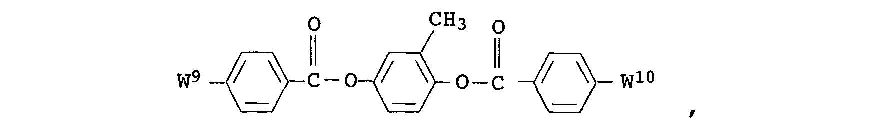 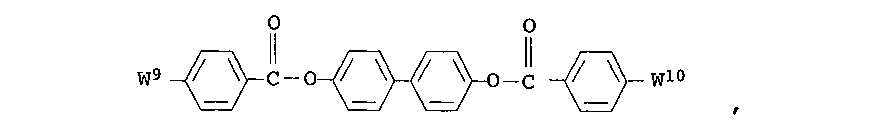 W⁹: CH₂=CH-C(=O)-O-(CH₂)₄-O-C(=O)-O- ,
W¹⁰: -O-(O=)C-O-(CH₂)₄-O-C(=O)-CH=CH₂ 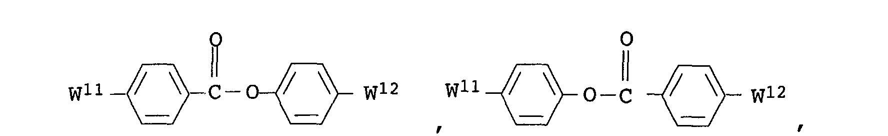 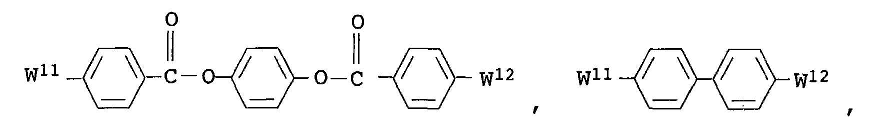 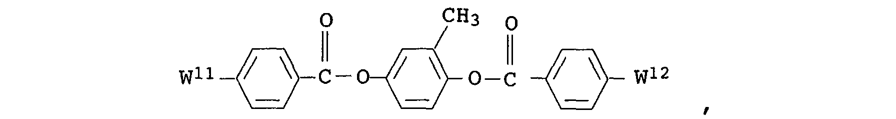 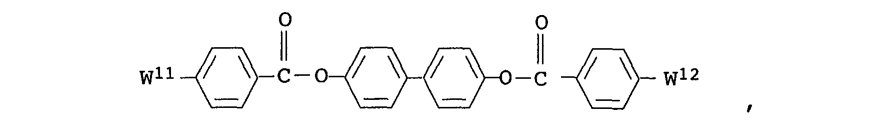 W¹¹: CH₂=CH-C(=O)-O-(CH₂)₆-O-C(=O)-O- ,
W¹²: -O-(O=)C-O-(CH₂)₆-O-C(=O)-CH=CH₂ 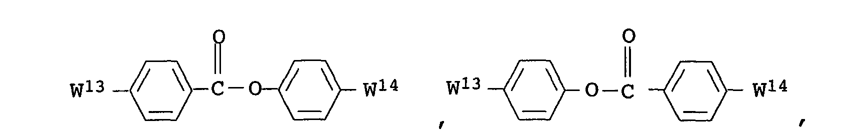 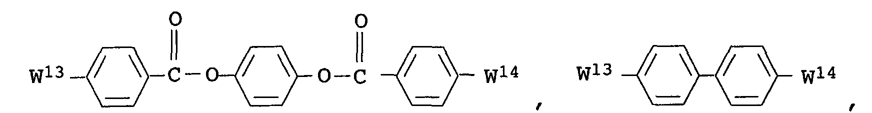 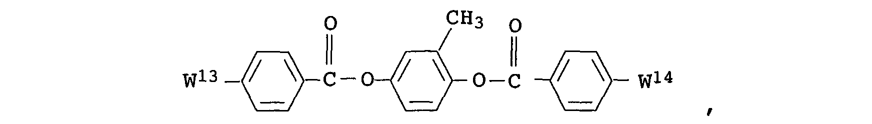 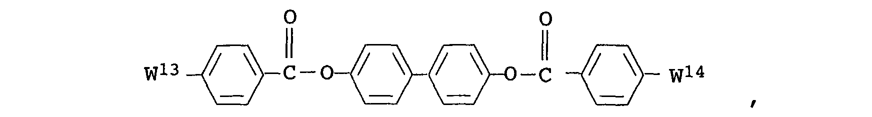 W¹³: CH₂=C(CH₃)-C(=O)-O-(CH₂)₄-O-C(=O)-O- ,
W¹⁴: -O-(O=)C-O-(CH₂)₄-O-C(=O)-C(CH₃)=CH₂ 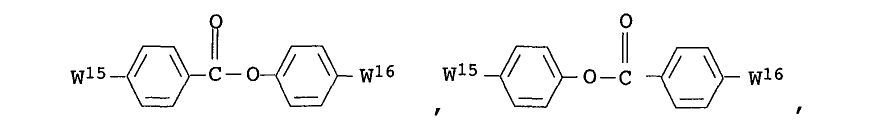 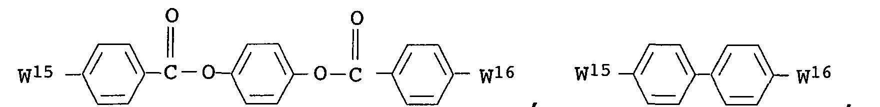 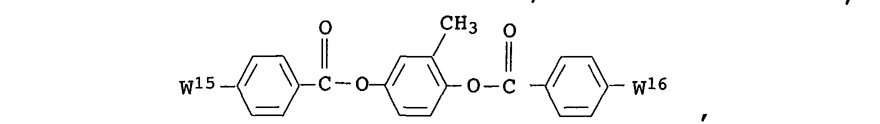 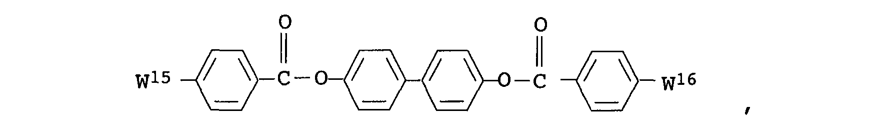 W¹⁵: CH₂=C(CH₃)-C(=O)-O-(CH₂)₆-O-C(=O)-O-,
W¹⁶: -O-(O=)C-O-(CH₂)₆-O-C(=O)-C(CH₃)=CH₂

Als besonders bevorzugte Monomere V sind folgende Strukturen zu nennen: 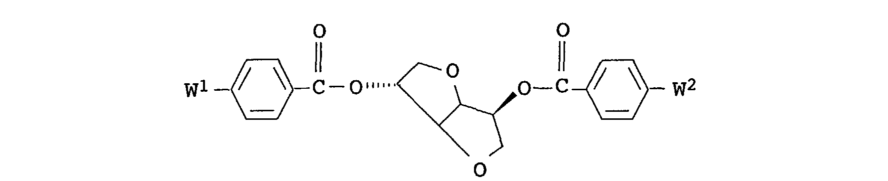 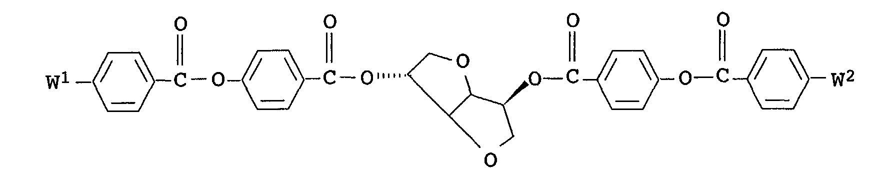 W¹: CH₂=CH-C(=O)-O-(CH₂)₄-O- W²: -O-(CH₂)₄-O-C(=O)-CH=CH₂ 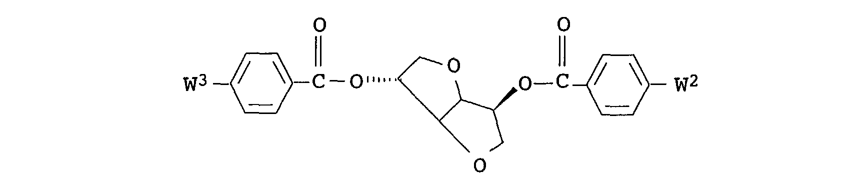 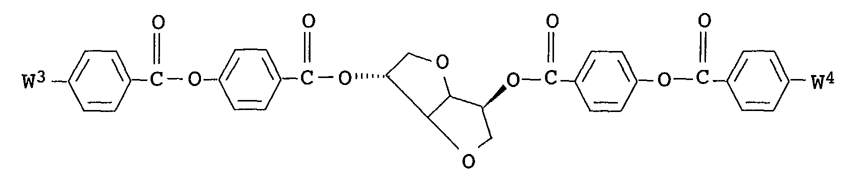 W³: CH₂=CH-C(=O)-O-(CH₂)₆-O- W⁴: -O-(CH₂)₆-O-C(=O)-CH=CH₂ 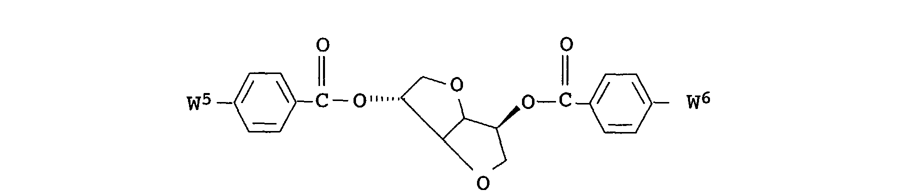 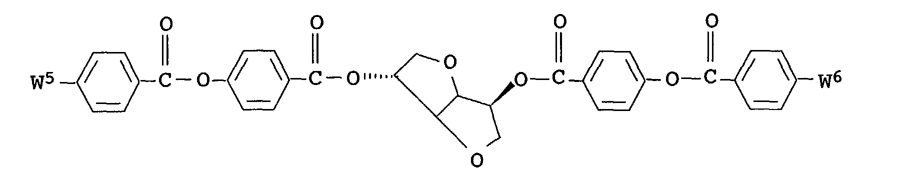 W⁵: CH₂=C(CH₃)-C(=O)-O-(CH₂)₄-O-, W⁶ : -O-(CH₂)₄-O-C(=O)-C(CH₃)=CH₂ 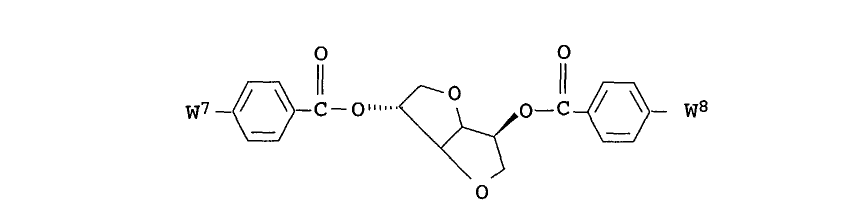 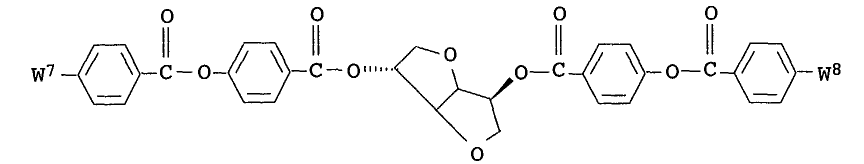 W⁷: CH₂=C(CH₃)-C(=O)-O-(CH₂)₆-O-, W⁸: -O-(CH₂)₆-O-C(=O)-C(CH₃)=CH₂ 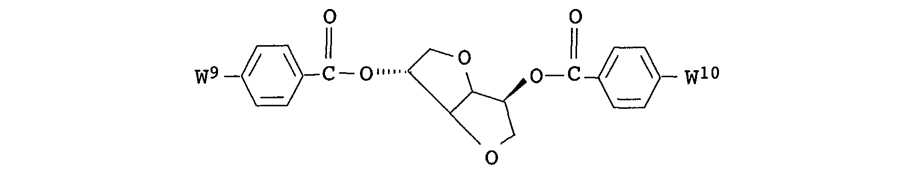 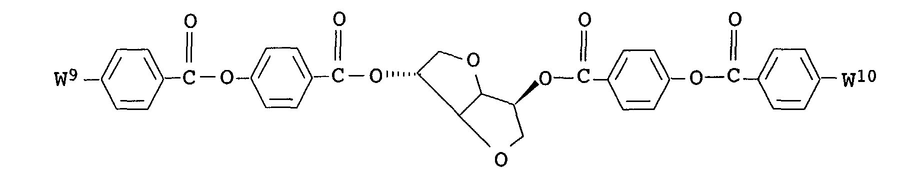 W⁹: CH₂=CH-C(=O)-O-(CH₂)₄-O-C(=O)-O-
W¹⁰: -O-(O=)C-O-(CH₂)₄-O-C(=O)-CH=CH₂ 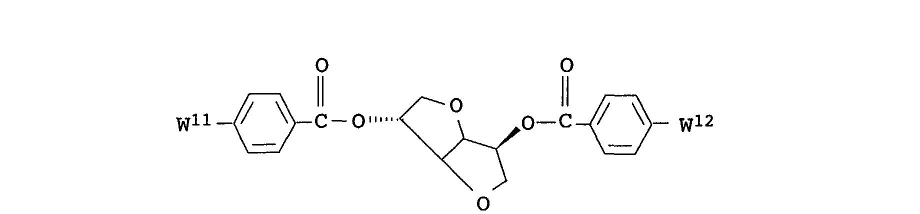 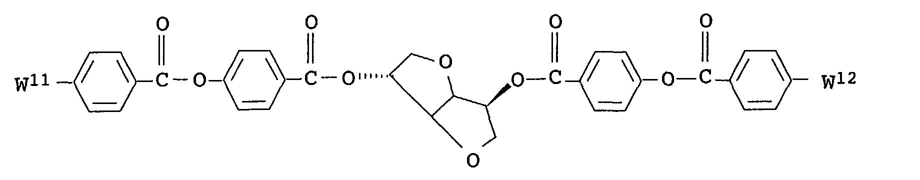 W¹¹: CH₂=CH-C(=O)-O-(CH₂)₆-O-C(=O)-O-
W¹²: -O-(O=)C-O-(CH₂)₆-O-C(=O)-CH=CH₂ 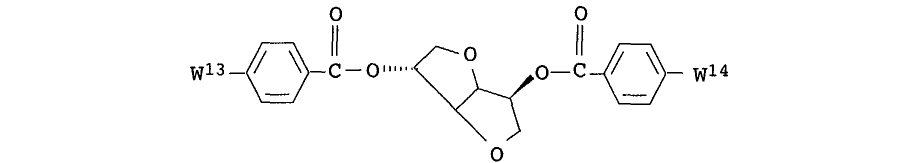 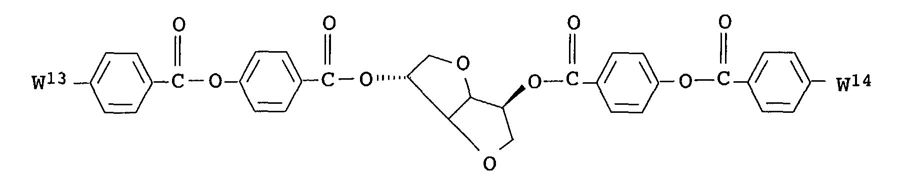 W¹³: CH₂=C(CH₃)-C(=O)-O-(CH₂)₄-O-C(=O)-O-,
W¹⁴: -O-(O=)C-O-(CH₂)₄-O-C(=O)-C(CH₃)=CH₂ 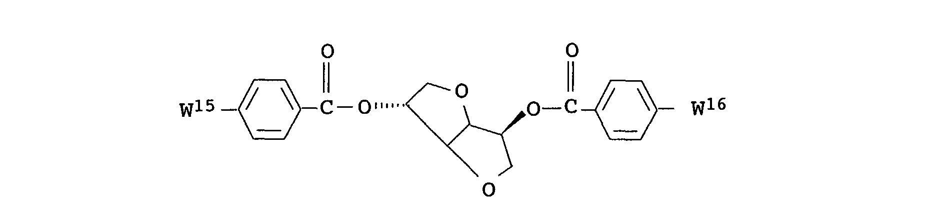 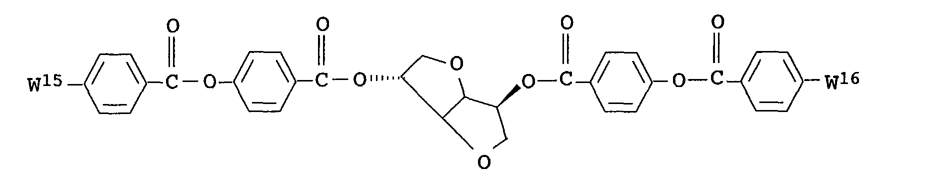 W¹⁵: CH₂=C(CH₃)-C(=O)-O-(CH₂)₆-O-C(=O)-O-,
W¹⁶: -O-(O=)C-O-(CH₂)₆-O-C(=O)-C(CH₃)=CH₂ 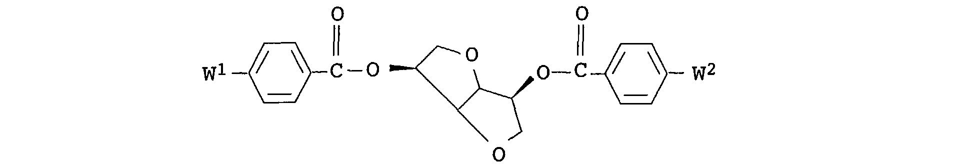 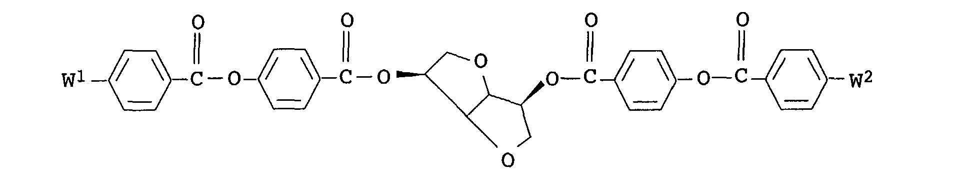 W¹: CH₂=CH-C(=O)-O-(CH₂)₄-O- W²: -O-(CH₂)₄-O-C(=O)-CH=CH₂ 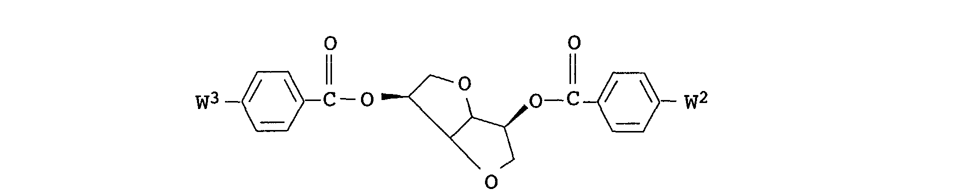 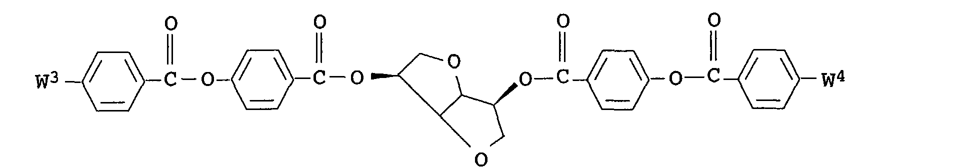 W³: CH₂=CH-C(=O)-O-(CH₂)₆-O- W⁴: -O-(CH₂)₆-O-C(=O)-CH=CH₂ 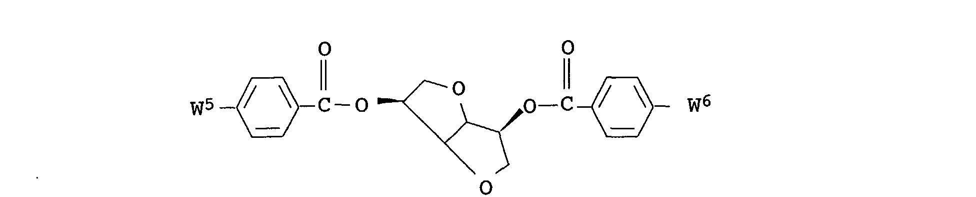 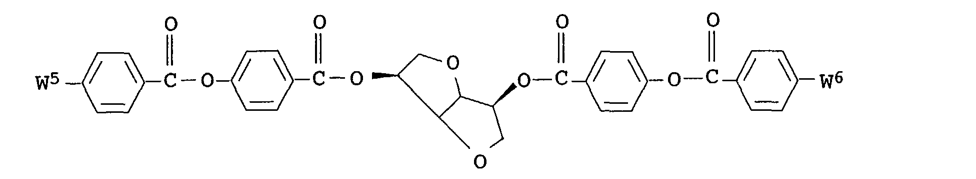 W⁵: CH₂=C(CH₃)-C(=O)-O-(CH₂)₄-O-, W⁶: -O-(CH₂)₄-O-C(=O)-C(CH₃)=CH₂ 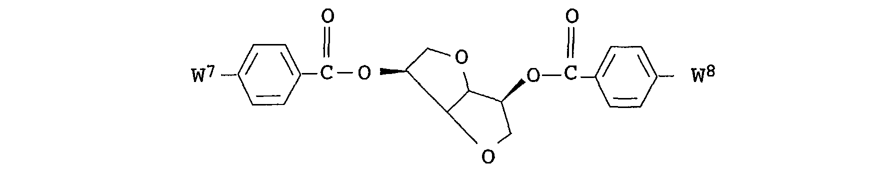 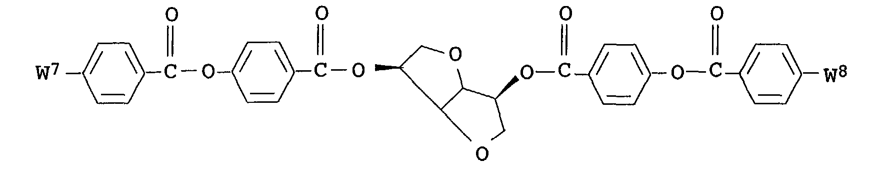 W⁷: CH₂=C(CH₃)-C(=O)-O-(CH₂)₆-O-, W⁸: -O-(CH₂)₆-O-C(=O)-C(CH₃)=CH₂ 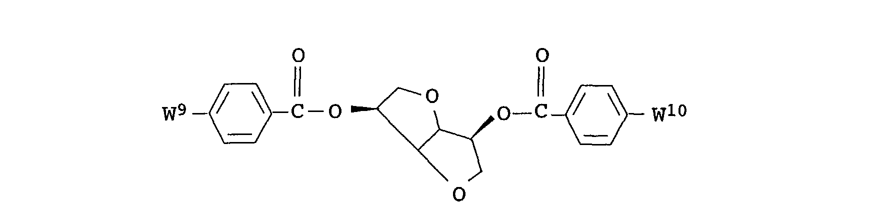 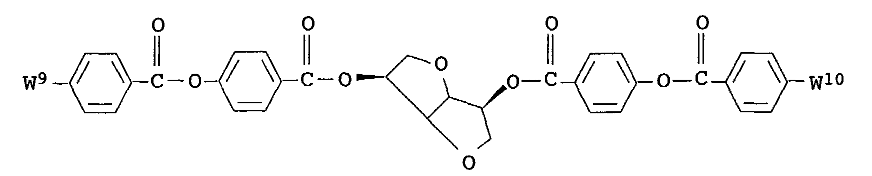 W⁹: CH₂=CH-C(=O)-O-(CH₂)₄-O-C(=O)-O-
W¹⁰: -O-(O=)C-O-(CH₂)₄-O-C(=O)-CH=CH₂ 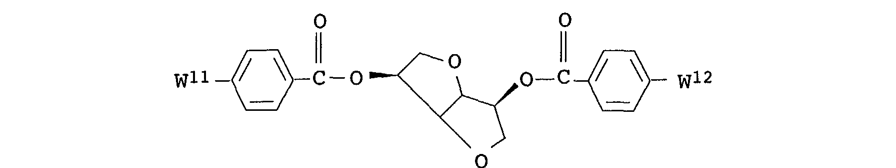 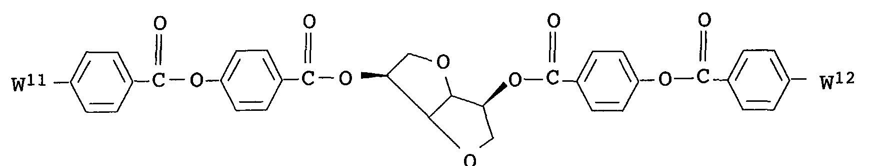 W¹¹: CH₂=CH-C(=O)-O-(CH₂)₆-O-C(=O)-O-
W¹²: -O-(O=)C-O-(CH₂)₆-O-C(=O)-CH=CH₂ 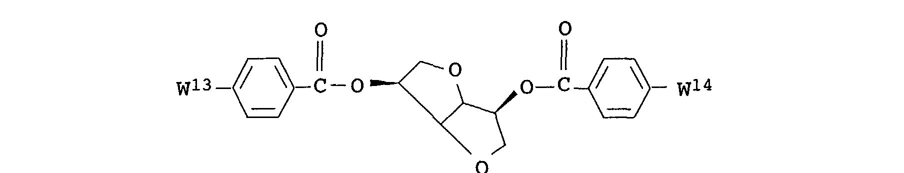 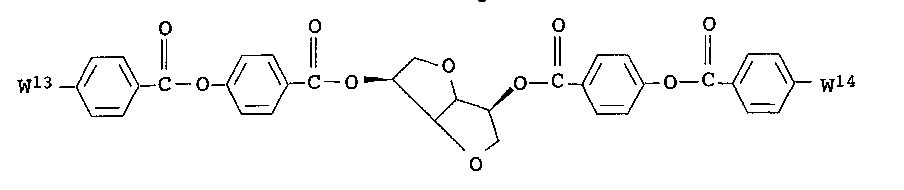 W¹³: CH₂=C(CH₃)-C(=O)-O-(CH₂)₄-O-C(=O)-O-,
W¹⁴: O-(O=)C-O-(CH₂)₄-O-C(=O)-C(CH₃)=CH₂ 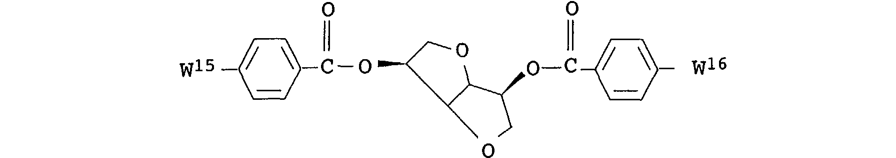 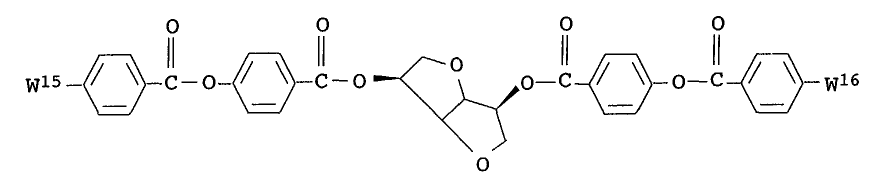 W¹⁵: CH₂=C(CH₃)-C(=O)-O-(CH₂)₆-O-C(=O)-O-,
W¹⁶: -O-(O=)C-O-(CH₂)₆-O-C(=O)-C(CH₃)=CH₂

Die Gewichtsverhältnisse von Komponente IV zu Komponente V liegen im Bereich von 99 zu 1 bis 40 zu 60, bevorzugt im Bereich von 99 zu 1 bis 70 zu 30, besonders bevorzugt von 98 zu 2 bis 85 zu 15.

Für die Herstellung der oben genannten Verbindungen der Varianten a) und b) sei auf die DE-A-198 24 972 verwiesen.

Bevorzugte, als Komponente B) geeignete, Polymere der Variante c) sind Polymere, die in ihren Polymerketten alternierend das Strukturelement -X-C(=O)- enthalten, worin X Sauerstoff oder NH bedeuten kann. Solche cholesterisch-flüssigkristalline Polymere sind beispielsweise in der DE-A-198 48 130 beschrieben, auf die hiermit in vollem Umfang Bezug genommen wird. Bevorzugte cholesterische Hauptgruppenpolymere sind aus folgenden Bausteinen der einzelnen Monomergruppen aufgebaut:
i) 1 bis 60 mol-%, bevorzugt 3 bis 50 mol-% mindestens einer chiralen, bifunktionellen Moleküleinheit;
ii) 0 bis 99 mol-%, bevorzugt 5 bis 90 mol-% mindestens einer achiralen Einheit aus der Gruppe der aromatischen Hydroxycarbonsäuren, cycloaliphatischen Hydroxycarbonsäuren, aromatischen Aminocarbonsäuren und cycloaliphatischen Aminocarbonsäuren;
iii) 0 bis 49,5 mol-%, bevorzugt 0 bis 40 mol-% mindestens einer achiralen Einheit aus der Gruppe der aromatischen Dicarbonsäuren und cycloaliphatischen Dicarbonsäuren und
iv) 0 bis 99 mol-%, bevorzugt 0 bis 49,5 mol-% mindestens einer achiralen Einheit aus der Gruppe der aromatischen Diole, cycloaliphatischen Diole, aromatischen Diamine und cycloaliphatischen Diamine,
v) 0 bis 5 mol-% einer Komponente mit mehr als zwei funktionellen Gruppen,
wobei die Summe der Einzelkomponenten i) bis v) 100 mol-% ergibt.

Die Komponenten aus der Gruppe i) rekrutieren sich zweckmäßig aus dem "chiralen pool". Hierunter wird in der Fachwelt (Ullmanns Encycl. Techn., 5. Auflage, Bd. A18, S. 183, 1991, VCH-Verlag) die Gesamtheit der natürlich vorkommenden chiralen Verbindungen verstanden. Insbesondere gehören hierzu sowohl die chiralen Bausteine tierischen als auch pflanzlichen Ursprungs. Dies schließt jedoch die Verwendung vollsynthetischer oder teilsynthetischer chiraler Molekülbausteine keinesfalls aus. So können auch aus Naturstoffen durch einen oder mehrere Syntheseschritte wertvolle chirale Komponenten erhalten werden, die so entweder nicht oder nur in geringen Mengen in der Natur vorkommen.

Insbesondere sind als Molekülbausteine i) generell alle chiralen, bifunktionellen Komponenten, beispielsweise chirale Diole oder Polyole, chirale Dicarbonsäuren, chirale Hydroxycarbonsäuren oder Aminocarbonsäuren gemeint.

Besonders bevorzugte chirale, bifunktionelle Monomere sind:

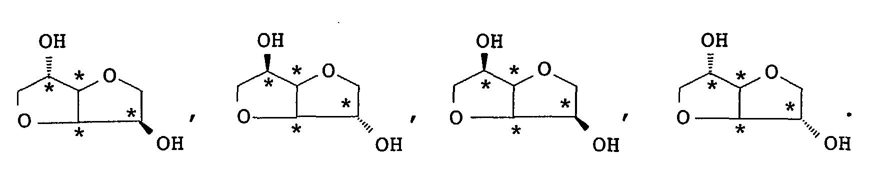

Die chiralen Comonomeren werden vorzugsweise in einer enantiomerenreinen Form eingesetzt. Bei Verwendung von Enantiomerengemischen eines Comonomeren ist darauf zu achten, daß eine Enantiomerenform in einem wirksamen Überschuss vorhanden ist.

Besonders bevorzugte Vertreter aus der Gruppe ii) sind:

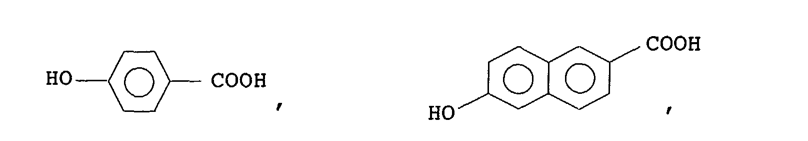

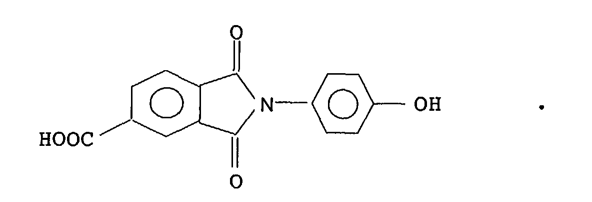

Besonders bevorzugte Vertreter aus der Gruppe iii) sind:

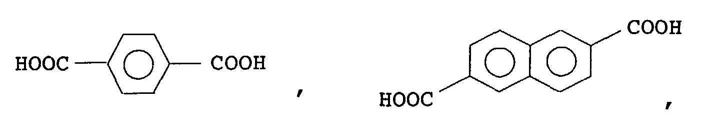

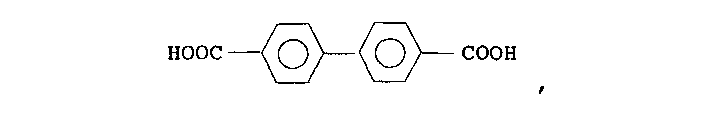

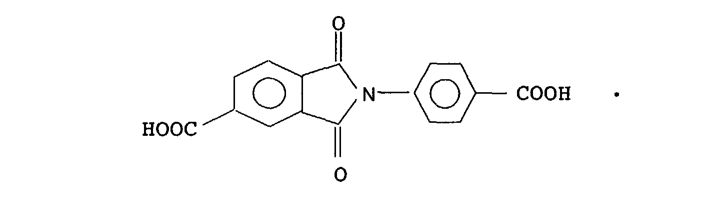

Besonders bevorzugte Vertreter aus der Gruppe iv) sind:

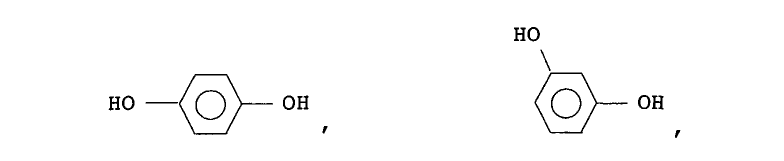

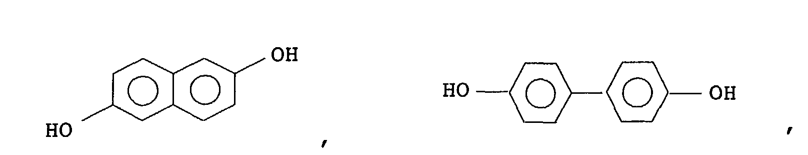

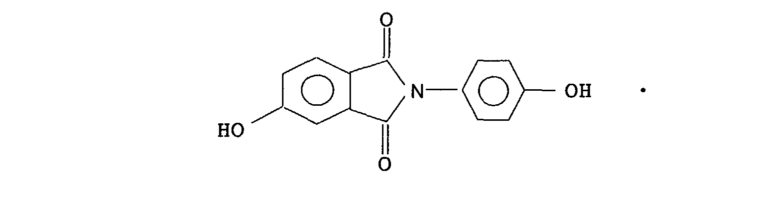

Anstelle der Carbonsäuren können auch andere dem Fachmann bekannte Carbonsäurederivate, wie beispielsweise Carbonsäurechloride, -anhydride oder -ester eingesetzt werden. Anstelle der Hydroxykomponenten können auch entsprechende Hydroxyderivate, wie z.B. die acylierten Hydroxyverbindungen eingesetzt werden.

Zusätzlich können die Polymere noch Komponenten mit mehr als zwei funktionellen Gruppen, wie beispielsweise Dihydroxybenzoesäure oder Trihydroxybenzole enthalten. Diese Komponenten wirken als Verzweigungsstelle im Polymer und werden gegebenenfalls nur in geringen Konzentrationen, beispielsweise 0 bis 5 mol-% zugegeben.

Die Herstellung dieser cholesterischen Polymeren ist in der DE-A-198 48 130 beschrieben.

Vorzugsweise wird das cholesterische Material des Bestandteils B) in Form von Pigmenten eingesetzt. Bezüglich der Herstellung von Pigmenten sei auf das zuvor Gesagte (siehe Bestandteil A)) verwiesen. Einzelheiten bezüglich der Herstellung cholesterisch-flüssigkristalliner Pigmente aus cholesterischen Polymeren sind der DE-A-198 48 130 zu entnehmen.

Die geeigneten und bevorzugten Korngrößen und Dicken der Pigmente liegen in dem zuvor für die Komponente A) beschriebenen Bereich.

Besonders bevorzugt enthält die erfindungsgemäße Zubereitung sogenannte UV-Breitbandreflektoren und UV-Mehrschichtreflektoren. Bezüglich deren Herstellung und Eigenschaften sei auf das zuvor für die Komponente A) Gesagte sowie auf die DE-A-198 24 972 verwiesen. Bevorzugt sind Dreischichtpigmente, bei denen die beiden äußeren Schichten aus jeweils einer gehärteten cholesterischen Komponente bestehen und die mittlere Schicht beispielsweise eine Bindemittelmatrix enthalten kann, in dem zusätzlich ein weiterer UV-Absorber eingearbeitet sein kann.

Der Anteil der cholesterischen Komponente in der vor ultravioletter Strahlung schützenden Komponente beträgt vorzugsweise 10 bis 90 Gew.-%, bevorzugt 20 bis 70 Gew.-%.

Als Komponente B) geeignete Aminobenzoesäuren und Aminobenzoesäureester sind beispielsweise p-Aminobenzoesäure, 2-Aminosäureester wie 5-Methyl-2-(1-methylethyl)-2-aminobenzoat, 4-Aminobenzoesäureester, wobei die Aminogruppe gegebenenfalls alkyliert oder alkoxiliert sein kann, z. B. 4-NH₂-C₆H₄-C(O)OCH(OH)CH₂OH, 4-Dimethylaminobenzoesäure-2-ethylhexylester, 4-Dimethylaminobenzoesäureisooctylester, 4-N(H₃CCH(OH)CH₂)₂-C₆H₄-C(O)OC₂H₅, 4-Bis(polyethoxy)paraaminobenzoesäurepolyethoxyethylester, 4-Aminobenzoesäure-1-glycerylester.

Als Komponente B) geeignete Bicyclo[2.2.1]heptan-2-one sind z. B. 3,3'-(1,4-Phenylendimethin)-bis(7,7-dimethyl-2-oxobicyclo[2.2.1]heptan-1-methansulfonsäure und ihre Salze, 3-(4'-Trimethylammonium)benzylidenbornan-2-on-methylsulfat, 3-(4'-Sulfo)benzylidenbornan-2-on und seine Salze, 3-Benzylidenbornan-2-on, 3-(4-Methylbenzyliden)campher.

Als Komponente B) geeignete Hydroxybenzoesäureester und -Salze sind Salicylsäureester wie z. B. 3,3,5-Trimethylcyclohexylsalicylat, 4-tert.-Butylphenylsalicylat, Salicylsäure-2-ethylhexylester, Salicylsäureisooctylester, 4-Isopropylbenzylsalicylat, und das Triethanolaminsalz sowie Digalloyltrioleat.

Als Komponente B) geeignete Benzophenone sind beispielsweise 2-Hydroxy-4-methoxybenzophenon, 2-Hydroxy-4-methoxybenzophenon-5-sulfonsäure und deren Salze, 2,2'-Dihydroxy-4-methoxybenzophenon (Dioxybenzone), 2-Hydroxy-4-methoxy-4-methylbenzophenon (Mexenon), 2,2',4,4'-Tetrahydroxybenzophenon, 2,4-Dihydroxybenzophenon, 2,2'-Dihydroxy-4,4'-dimethoxybenzophenon-5,5'-dinatriumsulfonat, Benzophenone-11 (Mischung tetrasubstituierter Benzophenone), 2,2'-Dihydroxy-4,4'-dimethoxybenzophenon, Verbindungen der Formel VI, 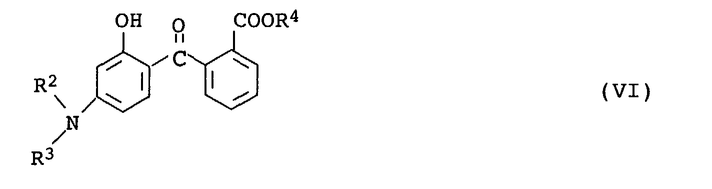 worin R² und R³ unabhängig voneinander Wasserstoff, C₁-C₂₀-Alkyl, C₃-C₁₀-Cycloalkyl oder C₃-C₁₀-Cycloalkenyl bedeuten, wobei die Substituenten R² und R³ gemeinsam mit dem Stickstoffatom, an das sie gebunden sind, einen 5- oder 6-Ring bilden können und R⁴ C₁-C₂₀-Alkyl, C₅-C₆-Cycloalkyl bedeutet.

Als Komponente B) geeignete Benzimidazole sind beispielsweise 2-Phenylbenzimidazol-5-sulfonsäure und deren Kalium-, Natriumund Triethanolaminsalze, 2,2'-(1,4-Phenylen)-bis-lH-benzimidazol-4,6-disulfonsäure oder deren Salze.

Als Komponente B) geeignete Zimtsäureester sind beispielsweise 4-Methoxyzimtsäure-2-isoamylester, 4-Methoxyzimtsäure-2-ethylhexylester.

Als Komponente B) geeignete 3,3-Diphenylacrylsäureester sind beispielsweise die der Formel VII, 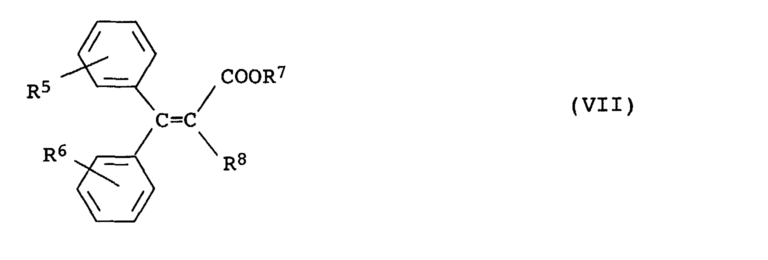 worin R⁵ und R⁶ unabhängig voneinander für Wasserstoff, lineares oder verzweigtes C₁-C₁₂-Alkyl, lineares oder verzweigtes C₁-C₁₂-Alkoxy, R⁷ für C₃-C₈-Cycloalkyl, das gegebenenfalls mit C₁-C₄-Alkylgruppen, insbesondere Methyl, Ethyl, n-Propyl, 1-Methylethyl, n-Butyl, 1-Methylpropyl, 2-Methylpropyl, 1,1-Dimethylethyl substituiert ist, und R⁸ für Wasserstoff oder CN stehen, insbesonders 2-Cyan-3,3-diphenylacrylsäurecyclopentylester, 2-Cyan-3,3-diphenylacrylsäurecyclohexylester, 2-Cyan-3,3-diphenylacrylsäure-4-tert.-butylcyclohexylester, 2-Cyan-3,3-diphenylacrylsäureethylhexylester.

Ein geeigneter 3-Imidazol-4-yl-acrylsäureester ist beispielsweise der Ethylester.

Eine als Komponente B) geeignete Phenylglyoxylsäure ist beispielsweise Dimethoxyphenylglyoxylsäure und ihr Natriumsalz.

Als Komponente B) geeignete Triazine sind beispielsweise 2,4,6-Triaryl-1,3,5-triazine, bei denen die Arylreste gegebenenfalls substituiert sind, z. B. 2,4,6-Trianilin-(o-carbo-2'-ethylhexyl-1'-oxy)-1,3,5-triazin, 2,4-bis-[4-(2-Ethylhexyloxy)-2-hydroxy]phenyl-6-(4-methoxyphenyl)-(1,3,5)-triazin sowie die weiteren folgende Verbindungen: 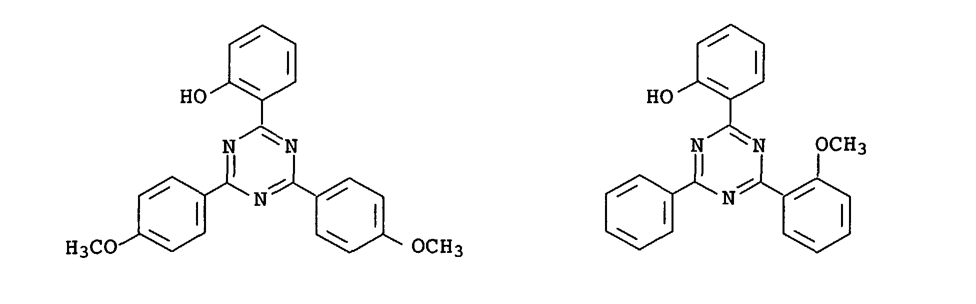 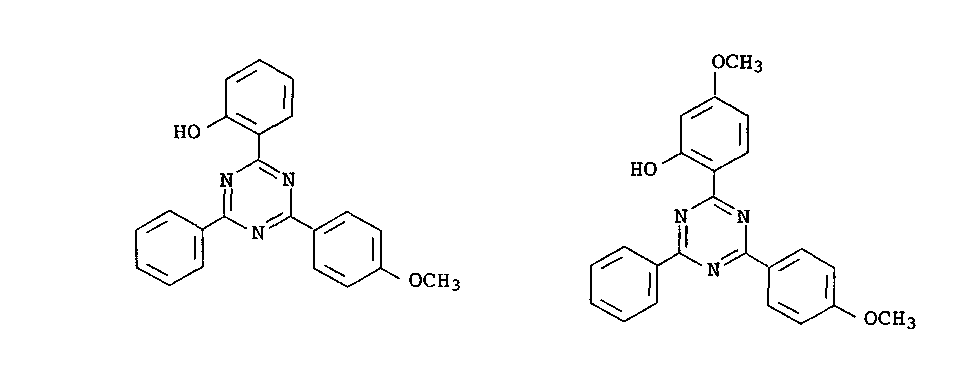 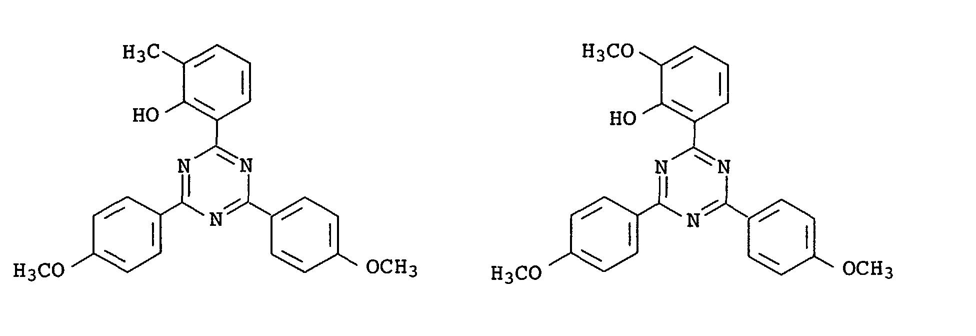 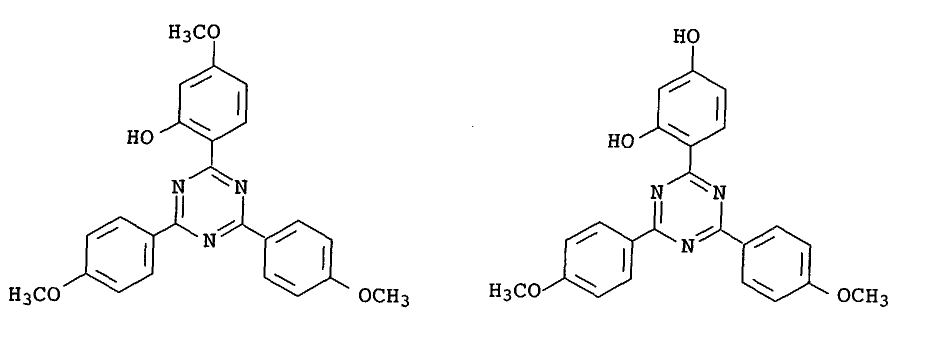 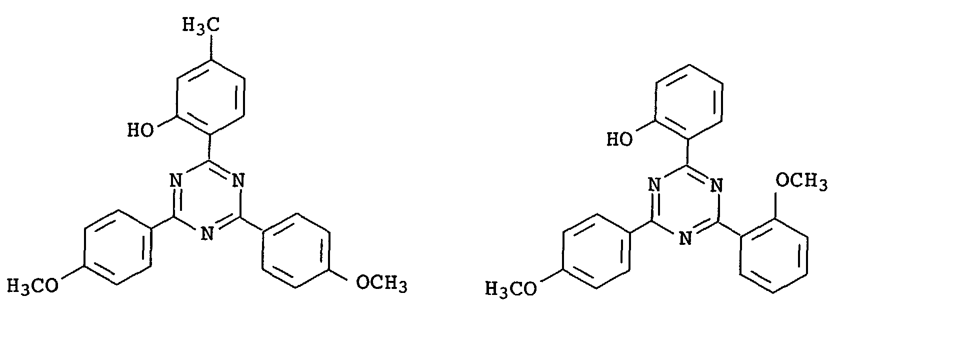 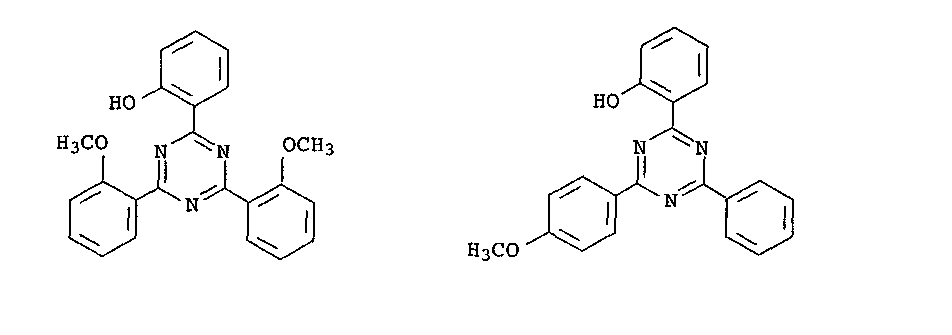 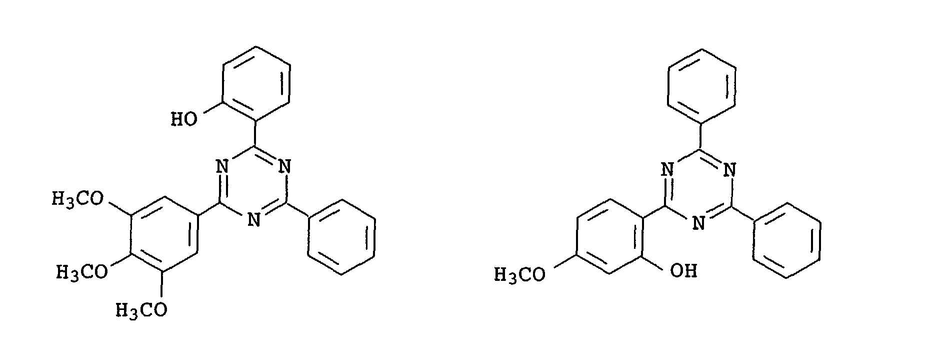 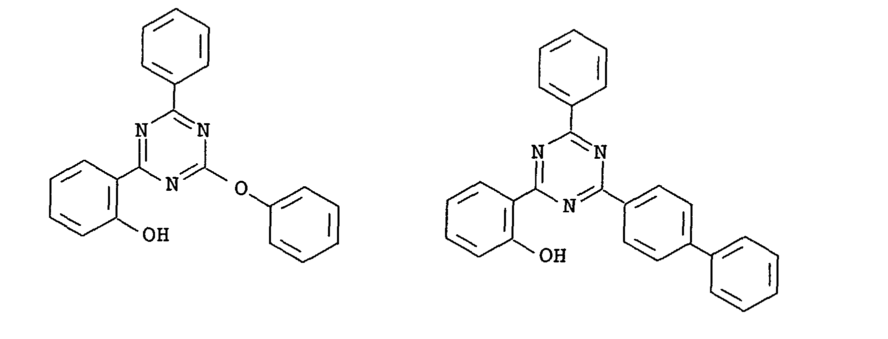 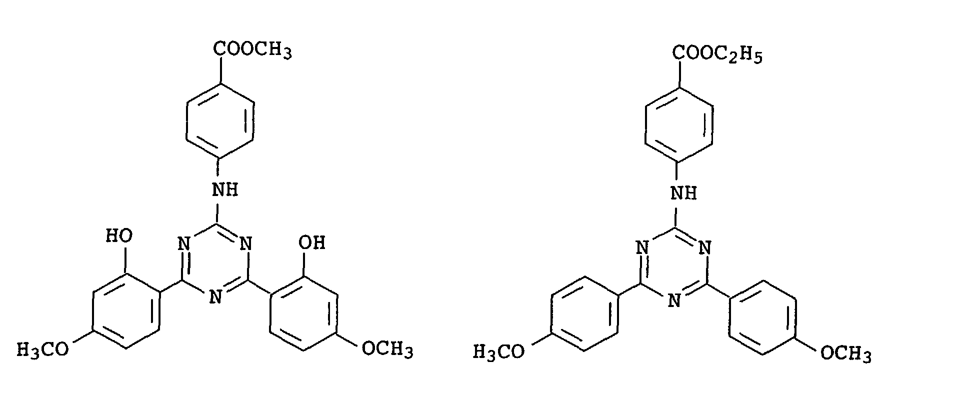 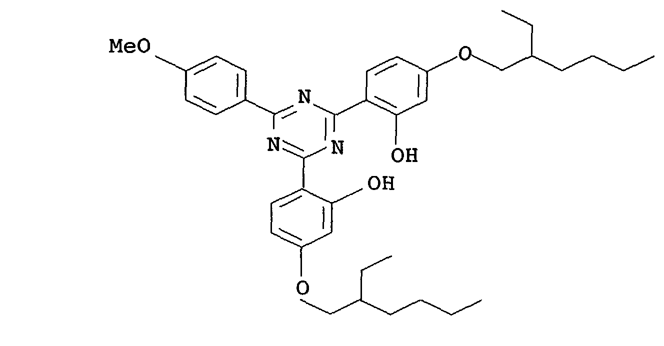 ferner Triazin-Derivate der Formel VIII, 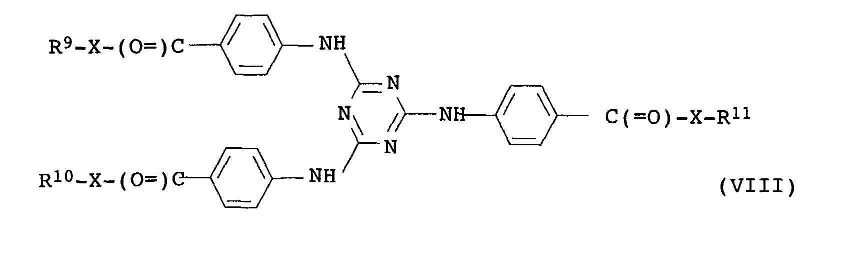 worin R⁹ bis R¹¹ unabhängig voneinander gegebenenfalls substituiertes C₁-C₂₀-Alkyl, C₅-C₁₀-Aryl, C₅-C₁₀-Heteroaryl oder SpSil bedeuten, wobei Sp für einen Spacer und Sil für einen Silan-, Oligosilan- oder Polysiloxanrest steht
X für die zweiwertigen Reste
―O― oder 〉N-R¹²
steht, wobei R¹² Wasserstoff oder gegebenenfalls substituiertes C₁-C₂₀-Alkyl, C₅-C₁₀-Aryl oder C₅-C₁₀-Heteroaryl bedeutet,
vorzugsweise

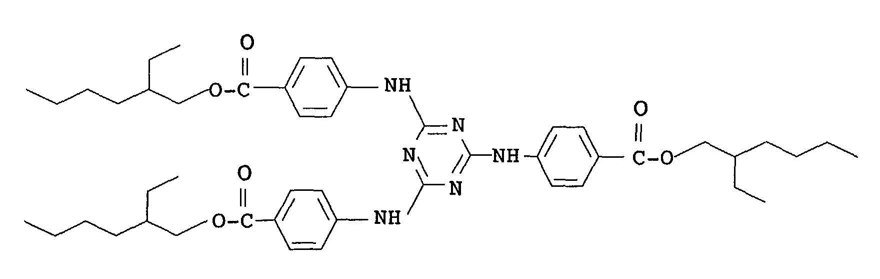

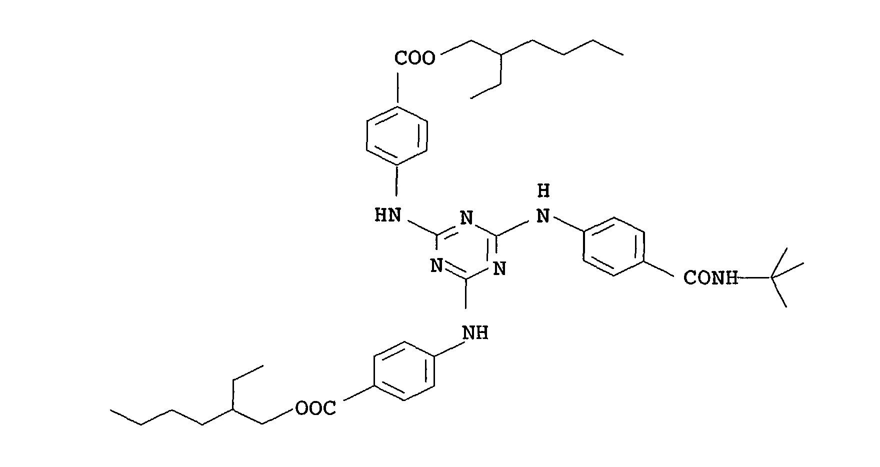

Als Komponente B) geeignete Dibenzoylmethane sind beispielsweise 2-Methyldibenzoylmethan, 4-Methyldibenzoylmethan, 4-Isopropyldibenzoylmethan, 4-tert.-Butyldibenzoylmethan, 2,4-Dimethyldibenzoylmethan, 2,5-Dimethyldibenzoylmethan, 4,4'-Diisopropyldibenzoylmethan, 4-tert.-Butyl-4'-methoxydibenzoylmethan, 2-Methyl-5-isopropyl-4'-methoxydibenzoylmethan, 2-Methyl-5-tert.butyl-4'-methoxydibenzoylmethan, 2,4-Dimethyl-4'-methoxydibenzoylmethan, 2,6-Dimethyl-4-tert.-butyl-4'-methoxydibenzoylmethan, insbesondere 4-tert.-Butyl-4'-methoxydibenzoylmethan.

Als Komponente B) geeignete 4,4'-Diarylbutadiene sind beispielsweise die der Formel IX, 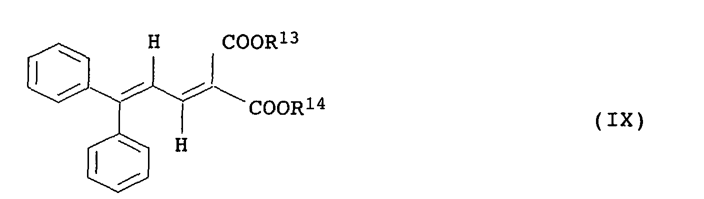 worin R¹³ und R¹⁴ unabhängig voneinander Wasserstoff, C₁-C₂₀-Alkyl, C₃-C₁₀-Cycloalkyl oder C₃-C₁₀-Cycloalkenyl bedeuten.

Als Komponente B) geeignete Benzotriazole sind beispielsweise

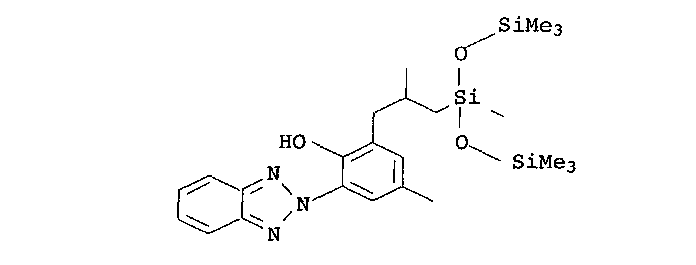

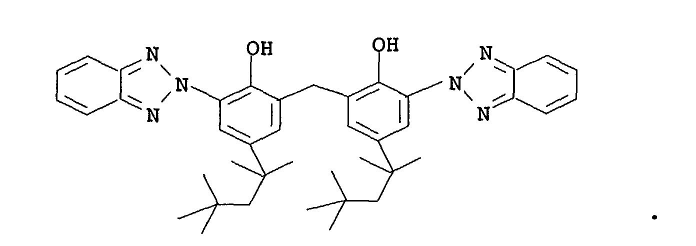

Als Komponente B) geeignete Organosiloxanbenzalmalonate sind beispielsweise die der Formel X , 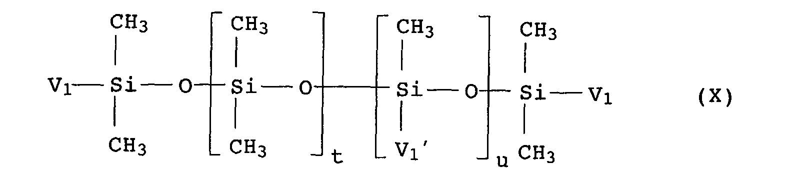 worin
V₁' die Gruppe 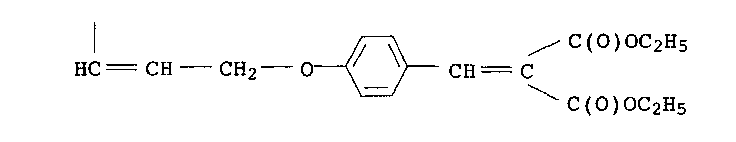 V₁ eine Methylgruppe oder V₁' bedeutet oder
der Formel XI 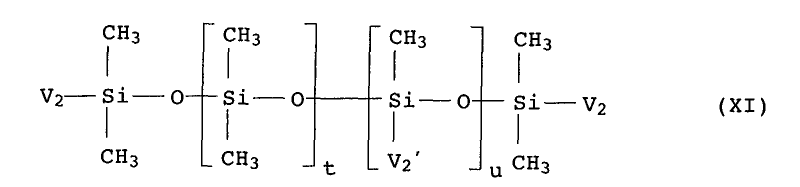 in der V₂' die Gruppe der Struktur 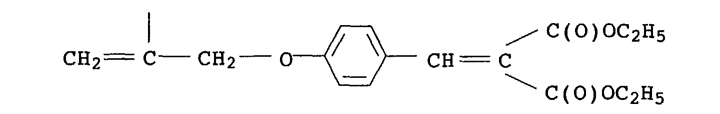 V₂ eine Methylgruppe oder V₂' bedeutet.

Als Komponente B) geeignetes Enamin ist beispielsweise

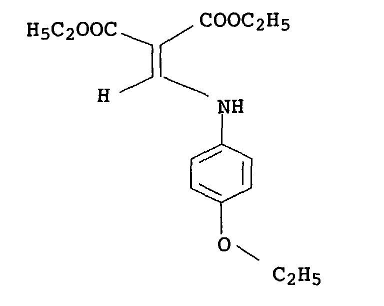

Als Komponente B) geeigneter cyclischer Iminoester ist beispielsweise

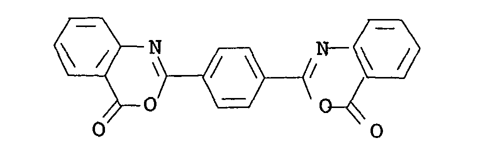

In einer bevorzugten Ausführungsform umfasst die Komponente B) eine Lichtschutzmittelkombination, die ein Hydroxybenzophenon-Derivat umfasst. Geeignete Kombinationen sind in der DE 100 12 408.9 beschrieben, auf die hiermit Bezug genommen wird.

In einer weiteren bevorzugten Ausführungsform umfasst die Komponente B) 3,3-Diphenylacrylate und Dibenzoylmethane. Geeignete Ausführungsformen sind in der DE 100 09 442.2 beschrieben, auf die hiermit Bezug genommen wird. Vorzugsweise umfasst die Komponente B weiterhin wenigstens einen 4-Methoxyzimtsäureester.

Bevorzugte, als Komponente B) geeignete anorganische Pigmente sind solche auf Basis von Metalloxiden und/oder anderen in Wasser schwerlöslichen oder unlöslichen Metallverbindungen, z. B. die Oxide des Titans (TiO₂), Zinks (ZnO), Eisens (z.B. Fe₂O₃), Zirkoniums (ZrO₂), Siliciums (SiO₂), Mangans (z. B. MnO), Aluminiums (Al₂O₃), Cers (z.B. Ce₂O₃), Mischoxide der entsprechenden Metalle sowie Abmischungen aus solchen Oxiden. Besonders bevorzugt handelt es sich um Pigmente auf der Basis Titandioxid und Zinkoxid.

Bevorzugt liegen die anorganischen Pigmente in hydrophober Form vor, d.h. sie sind oberflächlich wasserabweisend behandelt. Diese Oberflächenbehandlung kann darin bestehen, dass die Pigmente nach an sich bekannter Weise, wie in DE-A-33 14 742 beschrieben, mit einer dünnen hydrophoben Schicht versehen werden.

Vorteilhafte TiO₂-Pigmente sind beispielsweise unter den Handelsbezeichnungen MT 100 T von der Firma TAYCA, ferner M 160 von der Firma Kemira sowie T 805 von der Firma Degussa erhältlich.

Die kosmetischen oder dermatologischen Lichtschutzmittelzubereitungen enthalten vorteilhaft anorganische Pigmente, insbesondere Mikropigmente, in Mengen von z. B. 0,1 Gew.-% bis 30 Gew.-%, bevorzugt in Mengen von 0,5 Gew.-% bis 15 Gew.-%, besonders bevorzugt 1 Gew.-% bis 10 Gew.-%, ganz besonders bevorzugt 1,5 bis 6 Gew.-%, bezogen auf das Gesamtgewicht der Zubereitungen.

Die zuvor genannten Verbindungen des Bestandteils B) können einzeln, als Gemische jeweils einer Verbindungsklasse oder allgemein als Gemische eingesetzt werden. Schließlich können weitere, an sich bekannte, im UV-Bereich absorbierende/reflektierende Substanzen mitverwendet werden, sofern sie im Gesamtsystem der Zubereitung stabil sind.

Die Lichtschutzmittel enthaltenden kosmetischen und dermatologischen Zubereitungen enthalten einen Träger C), der unter Wasser, wassermischbaren Flüssigkeiten, hydrophoben Komponenten und Mischungen ausgewählt ist. Dazu zählen Wasser, C₁-C₄-Alkohole, wie Ethanol und Isopropanol, Fette, Wachse, Fettsäuren, Fettalkohole, Öle, Öl-in-Wasser- und Wasser-in-Öl-Emulsionen, Cremes und Pasten, Lippenschutzstiftmassen oder fettfreie Gele.

Als Emulsionen kommen u.a. auch O/W-Makroemulsionen, O/W-Mikroemulsionen oder O/W/O-Emulsionen in Frage, wobei die Emulsionen durch Phaseninversionstechnologie, z. B. gemäß DE-A-197 26 121 erhältlich sind.

Die hydrophobe Komponente (Lipidphase) kann vorteilhaft gewählt werden aus folgender Substanzgruppe:
- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z. B. Isopropanol, Propylenglykol oder Glycerin, oder Ester von Fettalkoholen mit Alkansäuren niedriger C-Zahl oder mit Fettsäuren; Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesätttigten und/oder ungesättigten, verzweigten und/oder unverzweigten Alkancarbonsäuren einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können vorteilhaft gewählt werden aus der Gruppe Isopropylmyristat, Isopropylpalmitat, Isopropylstearat, Isopropyloleat, n-Butylstearat, N-Hexyllaurat, N-Decyloleat, Iosoctylstearat, Isononylstearat, Isononylisononanoat, 2-Ethylhexylpalmitat, 2-Ethylhexyllaurat, 2-Hexyldecylstearat, 2-Octyldodecylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Siliconöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wqchse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂-C₁₅-Alkylbenzoat, Capryl-Caprin-triglycerid, Dicaprylether.

Besonders vorteilhaft sind Mischungen aus C₁₂-C₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisonononoat, aus Cyclomethicon und 2-Ethylhexylisostearat.

Die wässrige Phase der erfindungsgemäßen Zubereitung enthält gegebenenfalls vorteilhaft:
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl-, oder monoethylether und analoge Produkte.

Die erfindungsgemäßen Zubereitungen können kosmetische Hilfsstoffe enthalten. Übliche kosmetische Hilfsstoffe, die als Zusätze in Betracht kommen können, sind z.B. Co-Emulgatoren, Stabilisatoren, Verdickungsmittel, biogene Wirkstoffe, Filmbildner, Duftstoffe, Farbstoffe, Perlglanzmittel, Konservierungsmittel, Pigmente, Elektrolyte (z.B. Magnesiumsulfat); Insektenrepellentien und pH-Regulatoren. Als Co-Emulgatoren kommen vorzugsweise bekannte W/O- und daneben auch O/W-Emulgatoren wie etwa Polyglycerinester, Sorbitanester oder teilveresterte Glyceride in Betracht. Als Stabilisatoren können Metallsalze von Fettsäuren wie z.B. Magnesium-, Aluminium- und/oder Zinkstearat eingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polyacrylate, Polyvinylalkohol und Polyvinylpyrrolidon. Unter biogenen Wirkstoffen sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservierungsmittel eignen sich beispielsweise Formaldehydlösung, p-Hydroxybenzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, veröffentlicht im Verlag Chemie, Weinheim, 1984, zusammengestellt sind. Diese Farbstoffe werden üblicherweise in Konzentration von 0,001 bis 0,1 Gew.-%, bezogen auf die gesamte Mischung, eingesetzt.

Die erfindungsgemäße Zubereitung kann zusätzlich noch wenigstens ein Insektenrepellent enthalten. Dazu gehören z. B. 2-Ethyl-1,3-hexandiol, 2-Ethyl-2,3-hexandiol, 4,5-bis-(2-butylen)-tetrahydro-2-furaldehyd, Dimethylphthalat, Di-n-propyl-isocin-chomeronat und N,N,-Diethyl-m-toluolamid. Gegebenenfalls mischt man sie in einer Menge bis zu 15 Gew.-%, vorzugsweise 5 bis 15 Gew.-%, bezogen auf das Gesamtgewicht der Zubereitung bei.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhafterweise werden die Antioxidantien gewählt aus der Gruppe, bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl-, und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximine, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und deren Derivate (z.B. Ascorbylpalmitat, Mg-Ascorbylphosphat, Ascorbylacetat), Tocopherol und Derivate (z.B. Vitamin-E-Acetat, Tocotrienol), Vitamin A und Derivate (Vitamin-A-Palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, Trans-Stilbenoxid).

Die Menge der oben genannten Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 1 bis 10 Gew.-%, bezogen auf das Gesamtgewicht der Zubereitung.

Sofern Vitamin E und/oder dessen Derivate das oder die Antioxidantien darstellen, ist es vorteilhaft, deren jeweilige Konzentrationen aus dem Bereich von 0,001 - 10 Gew.-%, bezogen auf das Gesamtgewicht der Formulierung, zu wählen. Sofern Vitamin A bzw. Vitamin-A-Derivate bzw. Carotine bzw. deren Derivate das oder die Antixoidantien darstellen, ist es vorteilhaft, deren jeweilige Konzentrationen aus dem Bereich von 0,001 - 10 Gew.-%, bezogen auf das Gesamtgewicht der Formulierung, zu wählen.

Die Herstellung der Zubereitung kann in an sich bekannter Weise, d.h. beispielsweise durch Heiß-, Kalt-, Heiß-Heiß/Kalt- bzw. PIT-Emulgierung erfolgen. Hierbei handelt es sich um ein rein mechanisches Verfahren, eine chemische Reaktion findet nicht statt.

Die erfindungsgemäße Lichtschutzmittelzubereitung enthält im Allgemeinen
1 bis 20 Gew.-%, vorzugsweise 7 bis 15 Gew.-% Komponente A),
1 bis 35 Gew.-%, vorzugsweise 5 bis 25 Gew.-% Komponente B) und

### Träger C) und Hilfsstoffe ad 100 Gew.-%.

Die Formulierungsgrundlage erfindungsgemäßer dermatologischer Formulierungen enthält bevorzugt pharmazeutisch akzeptable Hilfsstoffe. Pharmazeutisch akzeptabel sind die im Bereich der Pharmazie, der Lebensmitteltechnologie und angrenzenden Gebieten bekanntermaßen verwendbaren Hilfstoffe, insbesondere die in einschlägigen Arzneibüchern (z. B. DAB Ph. Eur. BP NF) gelisteten sowie andere Hilfsstoffe, deren Eigenschaften einer physiologischen Anwendung nicht entgegenstehen.

Geeignete Hilfsstoffe können sein: Gleitmittel, Netzmittel, emulgierende und suspendierende Mittel, konservierende Mittel, Antioxidantien, Antireizstoffe, Chelatbildner, Emulsionsstabilisatoren, Filmbildner, Gelbildner, Geruchsmaskierungsmittel, Harze, Hydrokolloide, Lösemittel, Lösungsvermittler, Neutralisierungsmittel, Permeationsbeschleuniger, Pigmente, quarternäre Ammoniumverbindungen, Rückfettungs- und Überfettungsmittel, Salben-, Creme- oder Öl-Grundstoffe, Silikonderivate, Stabilisatoren, Sterilantien, Treibmittel, Trocknungsmittel, Trübungsmittel, Verdikkungsmittel, Wachse, Weichmacher, Weißöle. Eine diesbezügliche Ausgestaltung beruht auf fachmännischem Wissen, wie sie beispielsweise in Fiedler, H.P. Lexikon der Hilfsstoffe für Pharmazie, Kosmetik und angrenzende Gebiete, 4. Aufl., Aulendorf: ECV-Editio-Kantor-Verlag, 1996, dargestellt sind.

Beispiele geeigneter dermatologischer Formulierungen sind Salben, Cremes, Hydrogele, Pasten, sowie flüssige Arzneiformen, wie Lösungen, Emulsionen, insbesondere Öl-in-Wasser-Emulsionen, Suspensionen, beispielsweise Lotionen etc. Gewünschtenfalls können auch Liposomen oder Mikrosphären eingesetzt werden.

Zur Herstellung der erfindungsgemäßen dermatologischen Zubereitungen können Wirkstoffe mit einem geeigneten Hilfsstoff (Exzipient) vermischt oder verdünnt werden. Exzipienten können feste, halb feste oder flüssige Materialien sein, die als Vehikel, Träger oder Medium für den Wirkstoff dienen können. Die Zumischung weiterer Hilfsstoffe erfolgt gewünschtenfalls in der dem Fachmann bekannten Weise.

Gewünschtenfalls können mehrere Wirkstoffkomponenten gemeinsam formuliert werden. Sie können aber auch zunächst getrennt verarbeitet und anschließend in einer geeigneten Arzneiform zusammengeführt werden.

Zur Anwendung werden die erfindungsgemäßen kosmetischen oder dermatologischen Zubereitungen in der für die Kosmetika üblichen Weise auf die Haut und/oder die Haare in ausreichender Menge aufgebracht.

Die erfindungsgemäßen kosmetischen und/oder dermatologischen Lichtschutzformulierungen können dem kosmetischen und/oder dermatologischen Lichtschutz, ferner zur Behandlung, der Pflege und der Reinigung der Haut und/oder der Haare und als Schminkprodukt in der dekorativen Kosmetik dienen.

Besonders bevorzugt sind solche kosmetischen und dermatologischen Zubereitungen, die in der Form eines Sonnenschutzmittels vorliegen.

Solche Sonnenschutzpräparate können in flüssiger, pastöser oder fester Form vorliegen, beispielsweise als Wasser-in-Öl-Cremes, Öl-in-Wasser-Cremes und Lotionen, Aerosol-Schaumcremes, Gele, Öle, Fettstifte, Puder, Sprays, beispielsweise als Sprayöl oder Sprayemulsion oder alkoholisch-wässrige Lotionen.

Als Treibmittel für Aerosole kommen die üblichen Treibmittel in Frage, beispielhaft Propan, Butan, Pentan, Dimethylether und andere.

Zum Schutz menschlicher Haare vor UV-Strahlen können die erfindungsgemäß zu verwendeten Zubereitungen in Shampoos, Lotionen, Gele, Haarspray, Haarlack, Glanzspray, Haarstylingprodukten, Aerosol-Schaumcremes, Conditioner, Festigerlotionen, Fönlotionen, Fönfestiger oder Emulsionen eingearbeitet werden. Die jeweiligen Formulierungen können dabei u.a. zum Waschen, Färben sowie zum Frisieren der Haare verwendet werden.

Die erfindungsgemäßen Zubereitungen eignen sich hervorragend zum Schutz der menschliche Haut oder der menschlichen Haare vor Wärmestrahlung im infraroten Wellenlängenbereich von 750 bis 2500 nm und vor UV-Strahlung im Bereich von 280 bis 450 nm. Die Erwärmung der mit der erfindungsgemäßen Zubereitung behandelten Haut oder Haare ist im Vergleich zu unbehandelter oder nur mit UV-Lichtschutzmittel behandelter Haut und Haaren deutlich geringer. Die Reduzierung der Wärmestrahlung beträgt wenigstens 20 %, vorzugsweise wenigstens 30 %. Die Wärmestrahlung im infraroten Bereich des elektromagnetischen Spektrums wird weitestgehend reflektiert und nicht absorbiert, so dass sich Haut und Haare durch Wärmestrahlung nicht aufheizen. Die erfindungsgemäßen Zubereitungen zeichnen sich durch ein angenehmes Hautgefühl aus. Man fühlt sich frischer. Oberhalb 750 nm, insbesondere im Wellenlängenbereich von 751 bis 1600 nm werden mindestens 60 %, insbesondere 70 % und ganz besonders bevorzugt mindestens 80 % der auftreffenden Strahlung reflektiert.

Die folgenden Beispiele sollen die Erfindung näher erläutern ohne sie einzuschränken.

### I. Herstellung

Zur Herstellung der Pigmente 1 und 2 wurden als achirales, nematisches Monomer eine Verbindung der Formel 4 und als chiraler Dotierstoff die Verbindung der Formel 2B₁ bzw. 2B₂ eingesetzt. Die helikale Überstruktur der Pigmente, enthaltend die Verbindung 2B₁, war rechtsgängig und die helikale Überstruktur der Pigmente, enthaltend die Verbindung 2B₂, linksgängig.

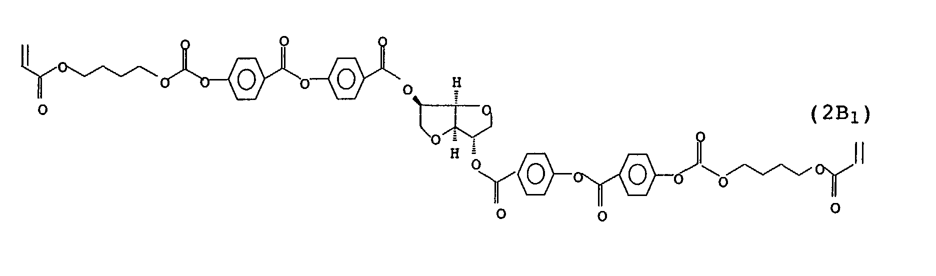

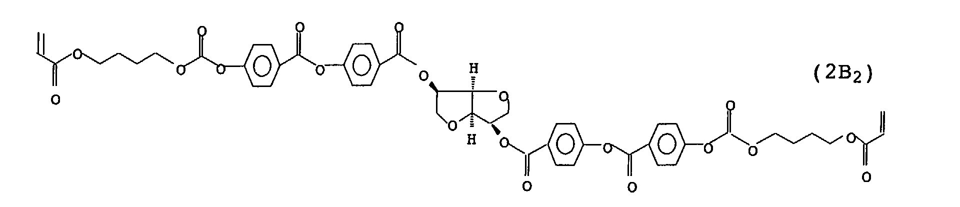

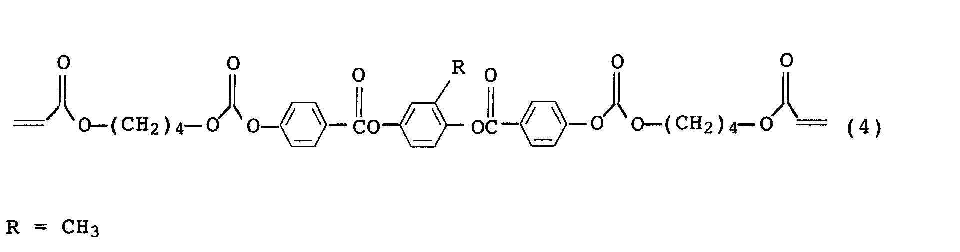

### Pigment 1: Reflexionsbereich 750 - 1200 nm

Zur Herstellung des Pigmentes 1 wurden acht cholesterische flüssigkristalline Mischungen verwendet, deren Zusammensetzungen sowie die Wellenlänge des Reflexionsmaximums λₘₐₓ jeder einzelnen Mischung der folgenden Tabelle 1 zu entnehmen ist.

**Tabelle 1:**

| Formel 2B₁ [Gew.-%] | Formel 2B₂ [Gew.-%] | Formel 4 [Gew.-%] | λₘₐₓ [nm] |
|---|---|---|---|
| 2,85 | - | 97,15 | 850 |
| 2,45 | - | 97,55 | 975 |
| 1,92 | - | 98,08 | 1200 |
| 1,65 | - | 98,35 | 1560 |
| - | 7,60 | 92,40 | 850 |
| - | 6,55 | 93,45 | 975 |
| - | 5,10 | 94,90 | 1200 |
| - | 4,10 | 95,90 | 1560 |

Zum Auftragen löste man die einzelnen Mischungen jeweils in Methylethylketon. Mittels eines Rakels wurden die acht Mischungen übereinander mit einer Feuchtfilmdicke von jeweils 2,5 µm einzeln nacheinander auf einer Polyethylenterephthalatfolie aufgebracht, das Lösungsmittel bei 70 °C abgezogen und der resultierende Film durch UV-Bestrahlung vernetzt und gehärtet. Die Beschichtung erfolgte nach einem in DE-A 196 38 797 beschriebenen Verfahren. Die helikale Überstruktur von vier Schichten war rechtsgängig, die der anderen vier Schichten linksgängig. Die gehärtete Multi-Cholesterenschicht wurde vom Träger abgelöst und durch Vermahlen und anschließendes Sieben klassiert. Die Korngröße der Pigmentpartikel lag im Bereich < 50 µm.

### Pigment 2: Reflexionsbereich 850 - 1600 nm

Zur Herstellung des Pigmentes 2 wurden sechs cholesterische Mischungen eingesetzt, deren Zusammensetzungen sowie die Wellenlänge des Reflexionsmaximums λₘₐₓ jeder Mischung Tabelle 2 zu entnehmen ist.

**Tabelle 2:**

| Formel 2B₁ [Gew.-%] | Formel 2B₂ [Gew.-%] | Formel 4 [Gew.-%] | λₘₐₓ [nm] |
|---|---|---|---|
| 2,45 | - | 97,55 | 975 |
| 1,92 | - | 98,08 | 1200 |
| 1,65 | - | 98,35 | 1560 |
| - | 6,55 | 93,45 | 975 |
| - | 5,10 | 94,90 | 1200 |
| - | 4,10 | 95,90 | 1560 |

Pigment 2 wurde in analoger Weise wie Pigment 1 hergestellt. Die helikale Überstruktur von drei Schichten war rechtsgängig, die der anderen drei Schichten linksgängig. Anschließend wurde jede Schicht zerkleinert und die Pigmente gemischt.

### II. Zubereitungen

### Beispiel 1

### Zusammensetzung für die Lippenpflege

| Massengehalt (Gew.-%) | |
|---|---|
| ad 100 | Eucerinum anhydricum |
| 10,00 | Glycerin |
| 10,00 | Titanium Dioxid, mikronisiert |
| 3,00 | 2-Cyan-3,3-diphenylacrylsäureethylhexylester |
| 2,00 | 4-tert.-Butyl-4'-methoxy-dibenzoylmethan |
| 8,00 | IR-Pigment 1 |
| 8,00 | Octyl Methoxycinnamat |
| 5,00 | Zink Oxid |
| 4,00 | Castoröl |
| 4,00 | Pentaerythrithyl Stearat/Caprat/Caprylat/Adipat |
| 3,00 | Glyceryl Stearat SE |
| 2,00 | Bienenwachs |
| 2,00 | Microkristallines Wachs |
| 2,00 | Quaternium-18 Bentonit |
| 1,50 | PEG-45/Dodecyl Glycol Copolymer |

### Beispiel 2

### Zusammensetzung für Sunblocker mit Mikropigmenten

| Massengehalt (Gew.-%) | |
|---|---|
| ad 100 | Wasser |
| 10,00 | Octyl Methoxycinnamat |
| 6,00 | PEG-7-Hydrogenated Castor Öl |
| 6,00 | Titanium Dioxid, mikronisiert |
| 3,00 | 2-Cyan-3,3-diphenylacrylsäureethylhexylester |
| 1,50 | 4-tert.-Butyl-4'-methoxy-dibenzoylmethan |
| 10,00 | IR-Pigment 2 |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

### Beispiel 3

### Fettfreies Gel

| Massengehalt (Gew.-%) | |
|---|---|
| ad 100 | Wasser |
| 8,00 | Octyl Methoxycinnamat |
| 7,00 | Titanium Dioxid, mikronisiert |
| 3,00 | 2-Cyan-3,3-diphenylacrylsäureethylhexylester |
| 1,50 | 4-tert.-Butyl-4'-methoxy-dibenzoylmethan |
| 13,00 | IR-Pigment 2 |
| 5,00 | Glycerin |
| 5,00 | PEG-25 PABA |
| 1,00 | 4-Methylbenzyliden Campher |
| 0,40 | Acrylate C₁₀-C₃₀ Alkyl Acrylat Crosspolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

### Beispiel 4

### Sonnencreme

| Massengehalt (Gew.-%) | |
|---|---|
| ad 100 | Wasser |
| 8,00 | Octyl Methoxycinnamat |
| 8,00 | Titanium Dioxid, mikronisiert |
| 6,00 | PEG-7-Hydrogenated Castor Öl |
| 3,00 | 2-Cyan-3,3-diphenylacrylsäureethylhexylester |
| 1,50 | 4-tert.-Butyl-4'-methoxy-dibenzoylmethan |
| 10,00 | IR-Pigment 1 |
| 6,00 | Mineral Öl |
| 5,00 | Zink Oxid |
| 5,00 | Isopropyl Palmitat |
| 0,30 | Imidazolidinyl Urea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

### Beispiel 5

### Sonnencreme wasserfest

| Massengehalt (Gew.-%) | |
|---|---|
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 3,00 | 2-Cyan-3,3-diphenylacrylsäureethylhexylester |
| 1,50 | 4-tert.-Butyl-4'-methoxy-dibenzoylmethan |
| 20,00 | IR-Pigment 2 |
| 4,00 | Glycerin |
| 3,00 | Jojoba Öl |
| 2,00 | 4-Methylbenzyliden Campher |
| 2,00 | Titanium Dioxid, mikronisiert |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

### Beispiel 6

### Tageslotion mit UV-Schutz

| Massengehalt (Gew.-%) | |
|---|---|
| ad 100 | Wasser |
| 2,00 | Cetearyl Alkohol |
| 1,00 | Glycerinmonostearat |
| 2,00 | Vaselin |
| 7,50 | Octyl Methoxycinnamat |
| 4,00 | Octyl Salicylat |
| 3,00 | 2-Cyan-3,3-diphenylacrylsäureethylhexylester |
| 1,50 | 4-tert.-Butyl-4'-methoxy-dibenzoylmethan |
| 11,00 | Pigment 1 |
| 0,50 | Dimethicon |
| 5,00 | Propylen Glycol |
| 0,20 | EDTA |
| 0,20 | Carbomer |
| 5,00 | C₁₂-C₁₅ Alkyl Benzoat |
| 0,27 | Triethanolamin |
| 1,00 | Tocopheryl Acetat |
| q.s. | Fragrance |

### Beispiel 7

### Tagescreme mit UV-Schutz

| Massengehalt (Gew.-%) | |
|---|---|
| ad 100 | Wasser |
| 2,00 | Cetearyl Alkohol |
| 2,00 | Cetyl Alkohol |
| 1,00 | Glycerinmonostearat |
| 2,00 | Vaselin |
| 7,50 | Octyl Methoxycinnamat |
| 4,00 | Octyl Salicylat |
| 3,00 | 2-Cyan-3,3-diphenylacrylsäureethylhexylester |
| 1,50 | 4-tert.-Butyl-4'-methoxy-dibenzoylmethan |
| 15,00 | Pigment 2 |
| 4,00 | Propylen Glycol |
| 0,20 | EDTA |
| 0,20 | Carbomer |
| 0,20 | Xanthan |
| 0,20 | C₁₀-C₃₀ Alkyl Acrylate Crosspolymer |
| 5,00 | C₁₂-C₁₅ Alkyl Benzoat |
| 0,54 | Triethanolamin |
| 1,00 | Tocopheryl Acetat |
| q.s. | Fragrance |
| q.s. | Konservierungsmittel |

### Beispiel 8

### Flüssiges Make Up

| Massengehalt (Gew.-%) | |
|---|---|
| ad 100 | Wasser |
| 2,00 | Cetearyl Alkohol |
| 2,00 | Ceteareth 25 |
| 6,00 | Glycerinmonostearat |
| 1,00 | Cetylalkohol |
| 8,00 | Paraffinöl |
| 7,00 | Cetearyl Octanoat |
| 0,2 | Dimethicon |
| 3,00 | Propylen Glycol |
| 1,00 | Panthenol |
| 3,00 | 2-Cyan-3,3-diphenylacrylsäureethylhexylester |
| 1,50 | 4-tert.-Butyl-4'-methoxy-dibenzoylmethan |
| 8,00 | Pigment 2 |
| 3,50 | Octyl Methoxycinnamat |
| 0,1 | Bisabolol |
| 5,70 | Titandioxid |
| 1,10 | Eisenoxid |
| q.s. | Fragrance |

### Beispiel 9

### Haargel mit Sonnenschutz

| Massengehalt (Gew.-%) | |
|---|---|
| ad 100 | Wasser |
| 1,20 | Carbomer |
| 0,50 | Hydroxyethyl Cellulose |
| 4,00 | Triethanolamin |
| 0,70 | PEG-40 Hydrogenated Castor oil |
| 1,50 | 2-Cyan-3,3-diphenylacrylsäureethylhexylester |
| 0,70 | 4-tert.-Butyl-4'-methoxy-dibenzoylmethan |
| 7,00 | Pigment 1 |
| 2,80 | Octyl Methoxycinnamat |
| 5,00 | Propylen Glycol |
| 0,01 | EDTA |
| q.s. | Fragrance |
| q.s. | Sicovit Patentblau 85 E 131 |

### III. Prüfung des Kühleffektes

### III.1 Zubereitung, enthaltend Pigment 1

Auf ein 6 cm² großes Prüffeld auf den Rücken eines Probanden trug man 12 mg der erfindungsgemäßen Zubereitung aus Beispiel 4 auf. Anschließend bestrahlte man die Hautstelle aus einer Entfernung von 4 cm mit einer Lampe OSRAM Longlife 64623 EVA 12V 100W. Die bestrahlte Fläche entsprach einem Kreis mit einem Durchmesser von 2 bis 3 cm. Man ermittelte sofort die Oberflächentemperatur der Haut mit einem Pyrometer "Ranger", AMIR 7814 der Fa. Ahlborn.

Zur Beurteilung des kühlenden Effekts wiederholte man den Versuch mit einem Sonnenschutzpräparat, das abgesehen von den IR-Reflektoren die gleiche Zusammensetzung aufwies. Ferner ermittelte man die Temperaturerhöhung auf unbehandelter Haut. Die Ergebnisse sind in der nachstehenden Tabelle 3 angegeben.

**Tabelle 3:**

| | Haut [°C] | Haut nach Behandlung mit UV-Lichtschutzmittel [°C] | Haut nach Behandlung mit erfindungsgemäßer Zubereitung [°C] |
|---|---|---|---|
| ohne Belichtung | 32,3 | 32,3 | 32,3 |
| nach 5 min, ohne Belichtung | 32,8 | 32,8 | 32,3 |
| nach 3 min Belichtung | 40,7 | 40,5 | 36,3 |
| Δ T | 7,9 | 7,7 | 4,0 |

Das mit der erfindungsgemäßen Zubereitung versehene Prüffeld zeigt eine deutlich geringere Temperaturerhöhung. Sie liegt um fast 50 % unter der Temperaturerhöhung von unbehandelter Haut . In dem Reflexionsbereich von 750 bis 1200 nm beobachtet man eine Reflexionsintensität von 90 %.

### III.2 Zubereitung, enthaltend Pigment 2

Die Prüfung des kühlenden Effektes von Pigment 2 erfolgte auf die in III.1 beschriebene Weise, nur trug man anstelle der erfindungsgemäßen Zubereitung aus Beispiel 4 12 mg der erfindungsgemäßen Zubereitung aus Beispiel 2 auf.

| | Haut [°C] | Haut nach Behandlung mit UV-Lichschutzmittel [oC] | Haut nach Behandlung mit erfindungsgemäßer Zubereitung [°C] |
|---|---|---|---|
| ohne Belichtung | 32,3 | 32,3 | 32,3 |
| nach 5 min, ohne Belichtung | 32,8 | 32,8 | 32,3 |
| nach 3 min Belichtung | 40,7 | 40,4 | 37,6 |
| Δ T | 7,9 | 7,6 | 5,3 |

Das mit der erfindungsgemäßen Zubereitung versehene Prüffeld zeigt eine deutlich geringere Temperaturerhöhung, die um 33 % unter der Temperaturerhöhung von unbehandelter Haut liegt. In dem Reflexionsbereich von 850 bis 1600 nm beobachtet man eine Reflexionsintensität von 85 %.

## Patentansprüche

1. Kosmetische oder dermatologische Zubereitung, enthaltend
A) wenigstens eine im infraroten Wellenlängenbereich von 750 nm bis 2500 nm reflektierende cholesterische Komponente,
B) wenigstens eine vor Strahlung im ultravioletten Strahlenbereich von 280 nm bis 449 nm schützende Komponente und
C) einen kosmetisch oder dermatologisch akzeptablen Träger.

2. Zubereitung nach Anpruch 1, wobei die Komponente A) ausgewählt ist unter
a) mindestens einem cholesterischen polymerisierbaren Monomer;
b) mindestens einem achiralen, nematischen, polymerisierbaren Monomer und einer chiralen Verbindung;
c) mindestens einem cholesterischen vernetzbaren Polymer;
d) mindestens einem cholesterischen Polymer in einem polymerisierbaren Verdünnungsmittel oder einem Gemisch polymerisierbarer Verdünnungsmittel;
e) mindestens einem cholesterischen Polymer, dessen cholesterische Phase durch schnelles Abkühlen unter die Glasübergangstemperatur eingefroren werden kann; oder
f) mindestens einem achiralem, flüssigkristallinen vernetzbaren Polymer und einer chirale Verbindung;
in gehärtetem Zustand.

3. Zubereitung nach einem der Ansprüche 1 oder 2, wobei die Komponente B) ausgewählt ist unter folgenden Klassen: cholesterischen Komponenten mit einer Ganghöhe kleiner 450 nm, Aminobenzoesäuren, Aminobenzoesäureestern, Bicyclo[2.2.1]heptan-2-onen, Hydroxybenzoesäureestern und -salzen, Benzophenonen, Benzimidazolen, Zimtsäureestern, 3,3-Diphenylacrylsäureestern, 3-Imidazol-4-yl-acrylsäuren, 3-Imidazol-4-yl-acrylsäureestern, alicyclischen Dienonen, Triazinen, Phenylglyoxylsäuren und -salzen, Dibenzoylmethanen, 4,4'-Diarylbutadienen, Benzotriazolen, Organosiloxanbenzalmalonaten, Enaminen, cyclischen Iminoestern, anorganischen Pigmenten und Mischungen davon.

4. Zubereitung nach Anspruch 3, wobei das anorganische Pigment ausgewählt ist unter Zinkoxid, Titanoxid und Gemischen davon.

5. Zubereitung nach einem der Ansprüche 3 oder 4, wobei die Komponente B) wenigstens ein 3,3-Diphenylacrylat und wenigstens ein Dibenzoylmethanderivat umfasst.

6. Zubereitung nach einem der Ansprüche 3 oder 4, wobei die Komponente B) wenigstens einen 4-Methoxyzimtsäureester enthält.

7. Zubereitung nach einem der vorhergehenden Ansprüche, wobei der Träger C) ausgewählt ist unter Wasser, wassermischbaren Flüssigkeiten, hydrophoben Komponenten und Mischungen davon.

8. Verwendung von kosmetischen oder dermatologischen Zubereitungen nach einem der Ansprüche 1 bis 7 zum Schutz der menschliche Haut oder der menschlichen Haare vor Wärmestrahlung im infraroten Wellenlängenbereich von 750 bis 2500 nm und vor UV-Strahlung im Bereich von 280 bis 449 nm.

9. Verwendung wenigstens einer im infraroten Wellenlängenbereich von 750 nm bis 2500 nm reflektierenden cholesterischen Komponente in kosmetischen oder dermatologischen Zubereitungen, die wenigstens eine vor Strahlung im ultravioletten Wellenlängenbereich von 280 bis 449 nm schützende Verbindung enthalten, zur Reduzierung einer Erwärmung der mit dieser Zubereitung behandelten Haut oder Haare.

10. Verwendung nach Anspruch 9, wobei die Reduzierung der Erwärmung mindestens 20 % beträgt.
